# EUROPEAN PATENT SPECIFICATION

(11) **EP 4 486 201 B1**
(45) Date of publication and mention of the grant of the patent: **04.11.2026**
(21) Application number: 23728456.7
(22) Date of filing: 17.04.2023
(51) Int. Cl.: A61B 5/0215, A61B 5/07, A61B 5/00

(54) **SENSOR IMPLANT DEVICE WITH STABILIZING APPENDAGE**
SENSORIMPLANTATVORRICHTUNG MIT STABILISIERENDEM ANHANG
DISPOSITIF D'IMPLANT DE CAPTEUR AVEC APPENDICE DE STABILISATION

(30) Priority: 18.04.2022 US 202263331963 P
(43) Date of publication of application: 08.01.2025
(73) Proprietor: Edwards Lifesciences Corporation, Irvine, CA 92614 (US)
(72) Inventor: VALDEZ, Michael, G., Irvine, CA 92614 (US)
(74) Representative: Venner, Julia Ann
(86) International application number: PCT/US2023/018829
(87) International publication number: WO 2023/205084

(56) References cited:
- US-A1- 2019 083 801
- US-A1- 2019 083 801
- US-A1- 2020 196 944
- US-A1- 2020 196 944
- US-A1- 2022 008 014
- US-A1- 2022 008 014

## Description

### RELATED APPLICATION

This application claims priority to U.S. Provisional Application No. 63/331,963, filed on April 18, 2022, entitled SENSOR IMPLANT DEVICE WITH STABILIZING APPENDAGE.

### BACKGROUND

The present disclosure generally relates to the field of a medical implant devices.

Various medical procedures involve the implantation of medical implant devices within the anatomy of the heart. Certain physiological parameters associated with such anatomy, such as fluid pressure, can have an impact on patient health prospects. Such devices are known from documents US 2020/196944 A1, US 2022/008014 A1, or US 2019/083801 A1.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention.

Described herein are one or more methods and/or devices to facilitate monitoring of physiological parameter(s) associated with certain chambers and/or vessels of the heart, such as the left atrium, using one or more sensor implant devices.

In accordance with some implementations of the present disclosure, a sensor implant device comprises a sensor body comprising at least a first sensor component, one or more anchors coupled to the sensor body and configured to anchor the sensor body within a tissue wall, a detachable tip, and a tether connecting the detachable tip to the sensor body.

The tether may be shape set to direct the detachable tip away from the sensor body. In some examples, the tether is configured to direct the detachable tip to a side of the sensor body.

In some examples, the tether is configured to direct the detachable tip towards a surface of the tissue wall. The tether may be composed a shape memory alloy.

The detachable tip may have a conical shape with a pointed end. In some examples, the pointed end is configured to pierce the tissue wall.

In some examples, the detachable tip has a rounded end. The detachable tip may be configured to provide a leading end for the sensor body during delivery to the tissue wall.

The detachable tip may comprise a flat base configured to lay flatly against a distal end of the sensor body.

In some examples, the one or more anchors comprise one or more proximal end anchors extending from a proximal end of the sensor body. The one or more proximal end anchors may have semi-circular shapes that may be concentric with the sensor body.

The one or more proximal end anchors may have semi-circular shapes that extend along the sensor body towards a distal end of the sensor body. In some examples, the one or more anchors comprise three proximal end anchors.

In some examples, the one or more anchors comprise one or more distal end anchors extending from a distal end of the sensor body.

The one or more distal end anchors may have semi-circular shapes that extend along the sensor body towards a proximal end of the sensor body. In some examples, the one or more anchors comprise three distal end anchors.

In some examples, the one or more anchors comprise one or more side anchors extending from a midsection of the sensor body. Each of the one or more side anchors may comprise a free end configured to form an approximately 45-degree angle with the sensor body.

The one or more anchors may comprise three side anchors. In some examples, the sensor implant device further comprises a marker attached to the sensor body.

In some examples, the sensor body comprises an inner lumen configured to receive a guidewire. The detachable tip may comprise a lumen configured to be aligned with the inner lumen of the sensor body during delivery.

Some implementations of the present disclosure relate to a method comprising delivering a shaft carrying a sensor implant device to a tissue wall. The sensor implant device comprises a sensor body comprising at least a first sensor component, one or more anchors coupled to the sensor body and configured to anchor the sensor body within a tissue wall, a detachable tip, and a tether connecting the detachable tip to the sensor body. The method further comprises retracting the shaft to allow the tether to pull the detachable tip away from the sensor body.

The tether may be shape set to direct the detachable tip away from the sensor body. In some examples, the tether is configured to direct the detachable tip to a side of the sensor body.

The tether may be configured to direct the detachable tip towards a surface of the tissue wall. In some examples, the tether is composed a shape memory alloy.

In some examples, the detachable tip has a conical shape with a pointed end. The pointed end may be configured to pierce the tissue wall.

The detachable tip may have a rounded end. In some examples, the detachable tip is configured to extend beyond a distal end of the shaft during delivery to the tissue wall.

In some examples, the detachable tip comprises a flat base configured to lay flatly against a distal end of the sensor body. The one or more anchors may comprise one or more proximal end anchors extending from a proximal end of the sensor body.

The one or more proximal end anchors may have semi-circular shapes that may be concentric with the sensor body. In some examples, the one or more proximal end anchors have semi-circular shapes that extend along the sensor body towards a distal end of the sensor body.

The one or more anchors may comprise three proximal end anchors.

In some examples, the one or more anchors comprise one or more distal end anchors extending from a distal end of the sensor body.

The one or more distal end anchors may have semi-circular shapes that extend along the sensor body towards a proximal end of the sensor body. In some examples, the one or more anchors comprise three distal end anchors.

In some examples, the one or more anchors comprise one or more side anchors extending from a midsection of the sensor body. Each of the one or more side anchors may comprise a free end configured to form an approximately 45-degree angle with the sensor body.

The one or more anchors may comprise three side anchors.

In some examples, the sensor implant device further comprises a marker attached to the sensor body. The sensor body may comprise an inner lumen configured to receive a guidewire.

The detachable tip may comprise a lumen configured to be aligned with the inner lumen of the sensor body during delivery.

For purposes of summarizing the disclosure, certain aspects, advantages, and novel features have been described. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting the scope of the inventions. In addition, various features of different disclosed examples can be combined to form additional examples, which are part of this disclosure. Throughout the drawings, reference numbers may be reused to indicate correspondence between reference elements.
Figure 1 illustrates an example representation of a human heart in accordance with one or more examples.
Figure 2 illustrates example pressure waveforms associated with various chambers and vessels of the heart according to one or more examples.
Figure 3 illustrates a graph showing left atrial pressure ranges.
Figure 4 is a block diagram representing an implant device in accordance with one or more examples.
Figure 5 is a block diagram representing a system for monitoring one or more physiological parameters associated with a patient according to one or more examples.
Figure 6 illustrates an example sensor assembly/device that can be a component of a sensor implant device, in accordance with one or more examples.
Figures 7A-7C illustrate a sensor implant device in accordance with one or more embodiments of the presently claimed invention.
Figures 8A and 8B illustrate another example sensor implant device in accordance with one or more embodiments of the presently claimed invention.
Figure 9 provides a perspective view of another example sensor implant device in accordance with one or more embodiments of the presently claimed invention.
Figure 10 illustrates a delivery system for delivering a sensor implant device of the presently claimed invention in accordance with one or more examples.
Figure 11 provides a side view of a sensor implant device in accordance with one or more embodiments of the presently claimed invention implanted and/or anchored within a heart in accordance with one or more examples.
Figure 12 (Figures 12-1 and 12-2) provides a flowchart illustrating a process including one or more steps for delivering one or more implants and/or sensors of the presently claimed invention to target locations within a heart, in accordance with one or more examples.
Figure 13 (Figures 13-1 and 13-2) provides images corresponding to steps of the process of Figure 12.

### DETAILED DESCRIPTION

The headings provided herein are for convenience only and do not necessarily affect the scope or meaning of the claimed invention.

Although certain preferred examples and examples are disclosed below, inventive subject matter extends beyond the specifically disclosed examples to other alternative examples and/or uses and to modifications and equivalents thereof. Thus, the scope of the claims that may arise herefrom is not limited by any of the particular examples described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain reference numbers are re-used across different figures of the figure set of the present disclosure as a matter of convenience for devices, components, systems, features, and/or modules having features that may be similar in one or more respects. However, with respect to any of the examples disclosed herein, re-use of common reference numbers in the drawings does not necessarily indicate that such features, devices, components, or modules are identical or similar. Rather, one having ordinary skill in the art may be informed by context with respect to the degree to which usage of common reference numbers can imply similarity between referenced subject matter. Use of a particular reference number in the context of the description of a particular figure can be understood to relate to the identified device, component, aspect, feature, module, or system in that particular figure, and not necessarily to any devices, components, aspects, features, modules, or systems identified by the same reference number in another figure. Furthermore, aspects of separate figures identified with common reference numbers can be interpreted to share characteristics or to be entirely independent of one another.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

The present disclosure relates to systems, devices, and methods for monitoring of one or more physiological parameters of a patient (e.g., blood pressure) using sensor-integrated cardiac shunts and/or other medical implant devices. In some implementations, the present disclosure relates to cardiac shunts and/or other cardiac implant devices that incorporate or are associated with pressure sensors or other sensor devices. The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly. Certain examples are disclosed herein in the context of cardiac implant devices. However, although certain principles disclosed herein are particularly applicable to the anatomy of the heart, it should be understood that sensor implant devices in accordance with the present disclosure may be implanted in, or configured for implantation in, any suitable or desirable anatomy.

While the various sensor devices described herein may be integrated with the various medical implant devices described herein, the sensor devices may be separate devices from the medical implant devices. For example, a sensor device may form a breakable and/or releasable connection with a medical implant device. Moreover, sensor devices described herein may be configured to be delivered separately (e.g., before and/or after) medical implant devices within a heart of a patient. For example, a sensor device may not be attached to a medical implant device during delivery processes (e.g., during delivery through a catheter) of the sensor device and/or medical implant device but may be attached/coupled to the medical implant device following delivery (e.g., following removal from a catheter) to a desired location within the heart. Example delivery locations can include the left atrium, the left atrial appendage, the pulmonary vein, the coronary sinus, and/or the various tissue walls associated with these locations.

In some examples, a catheter and/or guidewire used for delivering a sensor device may also be used for delivering a medical implant device. For example, the catheter and/or guidewire may remain within the body following delivery of the sensor device and/or medical implant device for delivery of the remaining device(s).

Examples described herein provide components for stabilizing and/or securely anchoring sensor devices within various anatomy. For example, a sensor device may comprise a detachable and/or detached leading end/tip configured to extend away from a senor body of the sensor device to provide an arm-like extension for the sensor body to lean on and/or be tethered to. In some examples, the detachable leading end may be configured to limit shaking of the sensor body and/or may be configured to improve synchronization of movement between the sensor body and a patient's heartbeat when anchored within a heart.

The terms "sensor device" and "sensor implant device" are used herein in accordance with their plain and ordinary meanings and may refer to any devices, implants, means for sensing, and/or other mechanisms configured to sense, measure, and/or determine various conditions within a patient's body. For example, a sensor device may be configured to measure blood flow velocity and/or pressure through one or more blood vessels and/or within a chamber of a heart. A sensor device may comprise a sensor component attached to and/or embedded within a sensor body and configured to perform various measurements.

The detachable leading end may be configured to contact a tissue wall and/or at least partially embed into the tissue wall. For example, the detachable leading end may comprise a pointed end configured to puncture the tissue wall, causing in-growth of tissue around the detachable leading end. In some examples, the detachable leading end/tip may comprise a coating to accelerate tissue in-growth around the detachable leading end/tip. A detachable tip may be configured to securely attach to and/or contact a sensor body of a sensor device. The terms "tip" and/or "leading end" are used in accordance with their plain and ordinary meanings and may refer to any means for leading, means for stabilizing, means for dilating, distal and/or proximal portion, cap, and/or other feature configured to be situated at or near a distal end of a sensor body. The detachable leading end/tip may be configured to dilate tissue during delivery into the body and/or to a target tissue wall.

In some examples, a sensor device may comprise one or more markers, including radiopaque markers, to facilitate visualization and/or location of the sensor device within a patient's body. A marker may be embedded within a sensor body of the device. In some examples, a tether interconnecting the detachable leading end/tip to the sensor body may be embedded into the sensor body and/or may be coupled to a marker. Contrast solution may be used to further facilitate visualization of the sensor device. The term "tether" is used herein in accordance with its plain and ordinary meaning and may refer to any cord, string, wire, means for tethering, and/or other device configured to form an attachment between a detachable and/or detached tip and a sensor body. The term "sensor body" is used herein in accordance with its plain and ordinary meaning and may refer to any portion of a sensor device configured to attach to components of the sensor device, which can include various anchors, tethers, and/or detachable tips.

### Cardiac Physiology

The anatomy of the heart is described below to assist in the understanding of certain inventive concepts disclosed herein. In humans and other vertebrate animals, the heart generally comprises a muscular organ having four pumping chambers, wherein the flow thereof is at least partially controlled by various heart valves, namely, the aortic, mitral (or bicuspid), tricuspid, and pulmonary valves. The valves may be configured to open and close in response to a pressure gradient present during various stages of the cardiac cycle (e.g., relaxation and contraction) to at least partially control the flow of blood to a respective region of the heart and/or to blood vessels (e.g., pulmonary, aorta, etc.).

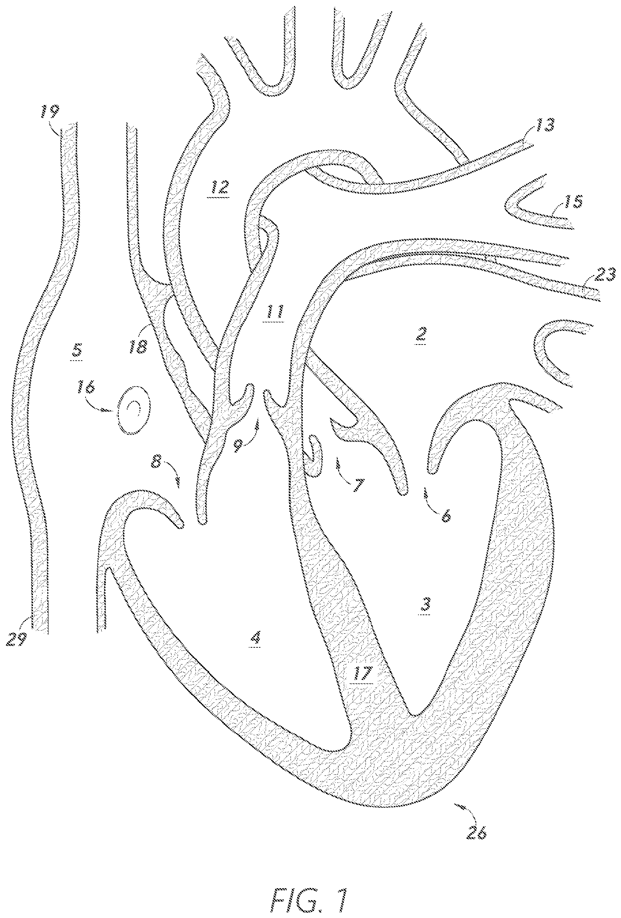

Figure 1 illustrates an example representation of a heart 1 having various features relevant to certain examples of the present inventive disclosure. The heart 1 includes four chambers, namely the left atrium 2, the left ventricle 3, the right ventricle 4, and the right atrium 5. In terms of blood flow, blood generally flows from the right ventricle 4 into the pulmonary artery 11 via the pulmonary valve 9, which separates the right ventricle 4 from the pulmonary artery 11 and is configured to open during systole so that blood may be pumped toward the lungs and close during diastole to prevent blood from leaking back into the heart from the pulmonary artery 11. The pulmonary artery 11 carries deoxygenated blood from the right side of the heart to the lungs. The pulmonary artery 11 includes a pulmonary trunk and left 15 and right 13 pulmonary arteries that branch off of the pulmonary trunk, as shown. The pulmonary veins 23 carry blood from the lungs to the left atrium 2.

In addition to the pulmonary valve 9, the heart 1 includes three additional valves for aiding the circulation of blood therein, including the tricuspid valve 8, the aortic valve 7, and the mitral valve 6. The tricuspid valve 8 separates the right atrium 5 from the right ventricle 4. The tricuspid valve 8 generally has three cusps or leaflets and may generally close during ventricular contraction (i.e., systole) and open during ventricular expansion (i.e., diastole). The mitral valve 6 generally has two cusps/leaflets and separates the left atrium 2 from the left ventricle 3. The mitral valve 6 is configured to open during diastole so that blood in the left atrium 2 can flow into the left ventricle 3, and, when functioning properly, closes during systole to prevent blood from leaking back into the left atrium 2. The aortic valve 7 separates the left ventricle 3 from the aorta 12. The aortic valve 7 is configured to open during systole to allow blood leaving the left ventricle 3 to enter the aorta 12, and close during diastole to prevent blood from leaking back into the left ventricle 3.

The heart valves may generally comprise a relatively dense fibrous ring, referred to herein as the annulus, as well as a plurality of leaflets or cusps attached to the annulus. Generally, the size of the leaflets or cusps may be such that when the heart contracts the resulting increased blood pressure produced within the corresponding heart chamber forces the leaflets at least partially open to allow flow from the heart chamber. As the pressure in the heart chamber subsides, the pressure in the subsequent chamber or blood vessel may become dominant and press back against the leaflets. As a result, the leaflets/cusps come in apposition to each other, thereby closing the flow passage. Dysfunction of a heart valve and/or associated leaflets (e.g., pulmonary valve dysfunction) can result in valve leakage and/or other health complications.

The atrioventricular (i.e., mitral and tricuspid) heart valves may further comprise a collection of chordae tendineae and papillary muscles (not shown) for securing the leaflets of the respective valves to promote and/or facilitate proper coaptation of the valve leaflets and prevent prolapse thereof. The papillary muscles, for example, may generally comprise finger-like projections from the ventricle wall. The valve leaflets are connected to the papillary muscles by the chordae tendineae. A wall of muscle, referred to as the septum, separates the left-side chambers from the right-side chambers. In particular, an atrial septum wall portion 18 (referred to herein as the "atrial septum," "interatrial septum," or "septum") separates the left atrium 2 from the right atrium 5, whereas a ventricular septum wall portion 17 (referred to herein as the "ventricular septum," "interventricular septum," or "septum") separates the left ventricle 3 from the right ventricle 4. The inferior tip 26 of the heart 1 is referred to as the apex and is generally located on or near the midclavicular line, in the fifth intercostal space.

The coronary sinus 16 comprises a collection of veins joined together to form a large vessel that collects blood from the heart muscle (myocardium). The ostium of the coronary sinus, which can be guarded at least in part by a Thebesian valve in some patients, is open to the right atrium 5, as shown. The coronary sinus runs along a posterior aspect of the left atrium 2 and delivers less-oxygenated blood to the right atrium 5. The coronary sinus generally runs transversely in the left atrioventricular groove on the posterior side of the heart.

Any of several access pathways in the heart 1 may be utilized for maneuvering guidewires and catheters in and around the heart 1 to deploy implants and/or devices of the present application. For instance, access may be from above via either the subclavian vein or jugular vein into the superior vena cava (SVC) 19, right atrium 5, and from there into the coronary sinus 16. Alternatively, the access path may start in the femoral vein and through the inferior vena cava (IVC) 14 into the heart 1. Other access routes may also be used, and each can utilize a percutaneous incision through which the guidewire and catheter are inserted into the vasculature, normally through a sealed introducer, and from there the physician can control the distal ends of the devices from outside the body.

### Health Conditions Associated with Cardiac Pressure and Other Parameters

As referenced above, certain physiological conditions or parameters associated with the cardiac anatomy can impact the health of a patient. For example, congestive heart failure is a condition associated with the relatively slow movement of blood through the heart and/or body, which causes the fluid pressure in one or more chambers of the heart to increase. As a result, the heart does not pump sufficient oxygen to meet the body's needs. The various chambers of the heart may respond to pressure increases by stretching to hold more blood to pump through the body or by becoming relatively stiff and/or thickened. The walls of the heart can eventually weaken and become unable to pump as efficiently. In some cases, the kidneys may respond to cardiac inefficiency by causing the body to retain fluid. Fluid build-up in arms, legs, ankles, feet, lungs, and/or other organs can cause the body to become congested, which is referred to as congestive heart failure. Acute decompensated congestive heart failure is a leading cause of morbidity and mortality, and therefore treatment and/or prevention of congestive heart failure is a significant concern in medical care.

The treatment and/or prevention of heart failure (e.g., congestive heart failure) can advantageously involve the monitoring of pressure in one or more chambers or regions of the heart or other anatomy. As described above, pressure buildup in one or more chambers or areas of the heart can be associated with congestive heart failure. Without direct or indirect monitoring of cardiac pressure, it can be difficult to infer, determine, or predict the presence or occurrence of congestive heart failure. For example, treatments or approaches not involving direct or indirect pressure monitoring may involve measuring or observing other present physiological conditions of the patient, such as measuring body weight, thoracic impedance, right heart catheterization, or the like. In some solutions, pulmonary capillary wedge pressure can be measured as a surrogate of left atrial pressure. For example, a pressure sensor may be disposed or implanted in the pulmonary artery, and readings associated therewith may be used as a surrogate for left atrial pressure. However, with respect to catheter-based pressure measurement in the pulmonary artery or certain other chambers or regions of the heart, use of invasive catheters may be required to maintain such pressure sensors, which may be uncomfortable or difficult to implement. Furthermore, certain lung-related conditions may affect pressure readings in the pulmonary artery, such that the correlation between pulmonary artery pressure and left atrial pressure may be undesirably attenuated. As an alternative to pulmonary artery pressure measurement, pressure measurements in the right ventricle outflow tract may relate to left atrial pressure as well. However, the correlation between such pressure readings and left atrial pressure may not be sufficiently strong to be utilized in congestive heart failure diagnostics, prevention, and/or treatment.

Additional solutions may be implemented for deriving or inferring left atrial pressure. For example, the E/A ratio, which is a marker of the function of the left ventricle of the heart representing the ratio of peak velocity blood flow from gravity in early diastole (the E wave) to peak velocity flow in late diastole caused by atrial contraction (the A wave), can be used as a surrogate for measuring left atrial pressure. The E/A ratio may be determined using echocardiography or other imaging technology; generally, abnormalities in the E/A ratio may suggest that the left ventricle cannot fill with blood properly in the period between contractions, which may lead to symptoms of heart failure, as explained above. However, E/A ratio determination generally does not provide absolute pressure measurement values.

Various methods for identifying and/or treating congestive heart failure involve the observation of worsening congestive heart failure symptoms and/or changes in body weight. However, such signs may appear relatively late and/or be relatively unreliable. For example, daily bodyweight measurements may vary significantly (e.g., up to 9% or more) and may be unreliable in signaling heart-related complications. Furthermore, treatments guided by monitoring signs, symptoms, weight, and/or other biomarkers have not been shown to substantially improve clinical outcomes. In addition, for patients that have been discharged, such treatments may necessitate remote telemedicine systems.

The present disclosure provides systems, devices, and methods for guiding the administration of medication relating to the treatment of congestive heart failure at least in part by directly monitoring pressure in the left atrium, or other chamber or vessel for which pressure measurements are indicative of left atrial pressure and/or pressure levels in one or more other vessels/chambers, such as for congestive heart failure patients in order to reduce hospital readmissions, morbidity, and/or otherwise improve the health prospects of the patient.

### Cardiac Pressure Monitoring

Cardiac pressure monitoring in accordance with examples of the present disclosure may provide a proactive intervention mechanism for preventing or treating congestive heart failure and/or other physiological conditions. Generally, increases in ventricular filling pressures associated with diastolic and/or systolic heart failure can occur prior to the occurrence of symptoms that lead to hospitalization. For example, cardiac pressure indicators may present weeks prior to hospitalization with respect to some patients. Therefore, pressure monitoring systems in accordance with examples of the present disclosure may advantageously be implemented to reduce instances of hospitalization by guiding the appropriate or desired titration and/or administration of medications before the onset of heart failure.

Dyspnea represents a cardiac pressure indicator characterized by shortness of breath or the feeling that one cannot breathe well enough. Dyspnea may result from elevated atrial pressure, which may cause fluid buildup in the lungs from pressure back-up. Pathological dyspnea can result from congestive heart failure. However, a significant amount of time may elapse between the time of initial pressure elevation and the onset of dyspnea, and therefore symptoms of dyspnea may not provide sufficiently-early signaling of elevated atrial pressure. By monitoring pressure directly according to examples of the present disclosure, normal ventricular filling pressures may advantageously be maintained, thereby preventing or reducing effects of heart failure, such as dyspnea.

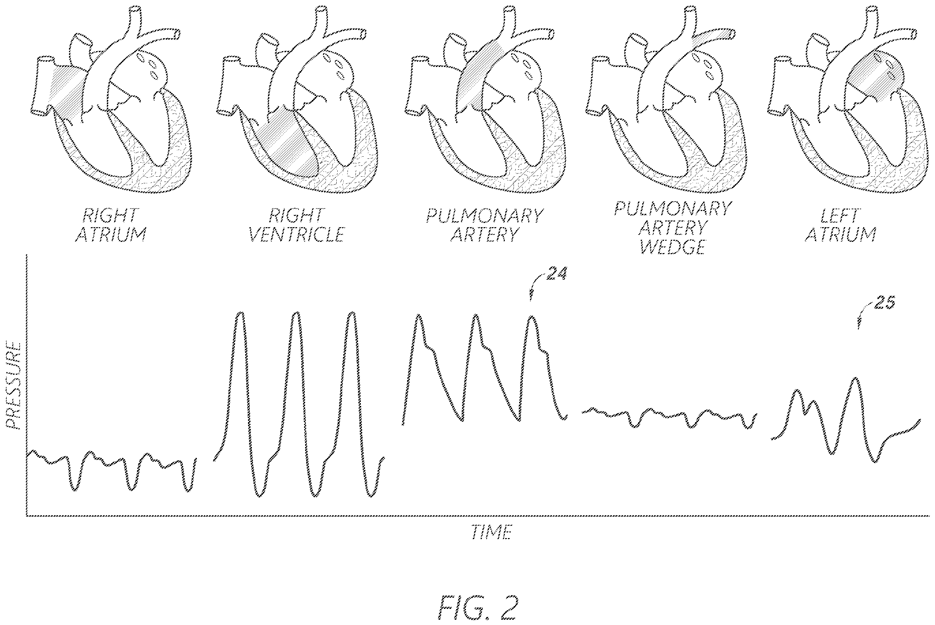

As referenced above, with respect to cardiac pressures, pressure elevation in the left atrium may be particularly correlated with heart failure. Figure 2 illustrates example pressure waveforms associated with various chambers and vessels of the heart according to one or more examples. The various waveforms illustrated in Figure 2 may represent waveforms obtained using right heart catheterization to advance one or more pressure sensors to the respective illustrated and labeled chambers or vessels of the heart. As illustrated in Figure 2, the waveform 25, which represents left atrial pressure, may be considered to provide the best feedback for early detection of congestive heart failure. Furthermore, there may generally be a relatively strong correlation between increases and left atrial pressure and pulmonary congestion.

Left atrial pressure may generally correlate well with left ventricular end-diastolic pressure. However, although left atrial pressure and end-diastolic pulmonary artery pressure can have a significant correlation, such correlation may be weakened when the pulmonary vascular resistance becomes elevated. That is, pulmonary artery pressure generally fails to correlate adequately with left ventricular end-diastolic pressure in the presence of a variety of acute conditions, which may include certain patients with congestive heart failure. For example, pulmonary hypertension, which affects approximately 25% to 83% of patients with heart failure, can affect the reliability of pulmonary artery pressure measurement for estimating left-sided filling pressure. Therefore, pulmonary artery pressure measurement alone, as represented by the waveform 24, may be an insufficient or inaccurate indicator of left ventricular end-diastolic pressure, particularly for patients with co-morbidities, such as lung disease and/or thromboembolism. Left atrial pressure may further be correlated at least partially with the presence and/or degree of mitral regurgitation.

Left atrial pressure readings may be relatively less likely to be distorted or affected by other conditions, such as respiratory conditions or the like, compared to the other pressure waveforms shown in Figure 2. Generally, left atrial pressure may be significantly predictive of heart failure, such as up two weeks before manifestation of heart failure. For example, increases in left atrial pressure, and both diastolic and systolic heart failure, may occur weeks prior to hospitalization, and therefore knowledge of such increases may be used to predict the onset of congestive heart failure, such as acute debilitating symptoms of congestive heart failure.

Cardiac pressure monitoring, such as left atrial pressure monitoring, can provide a mechanism to guide administration of medication to treat and/or prevent congestive heart failure. Such treatments may advantageously reduce hospital readmissions and morbidity, as well as provide other benefits. An implanted pressure sensor in accordance with examples of the present disclosure may be used to predict heart failure up two weeks or more before the manifestation of symptoms or markers of heart failure (e.g., dyspnea). When heart failure predictors are recognized using cardiac pressure sensor examples in accordance with the present disclosure, certain prophylactic measures may be implemented, including medication intervention, such as modification to a patient's medication regimen, which may help prevent or reduce the effects of cardiac dysfunction. Direct pressure measurement in the left atrium can advantageously provide an accurate indicator of pressure buildup that may lead to heart failure or other complications. For example, trends of atrial pressure elevation may be analyzed or used to determine or predict the onset of cardiac dysfunction, wherein drug or other therapy may be augmented to cause reduction in pressure and prevent or reduce further complications.

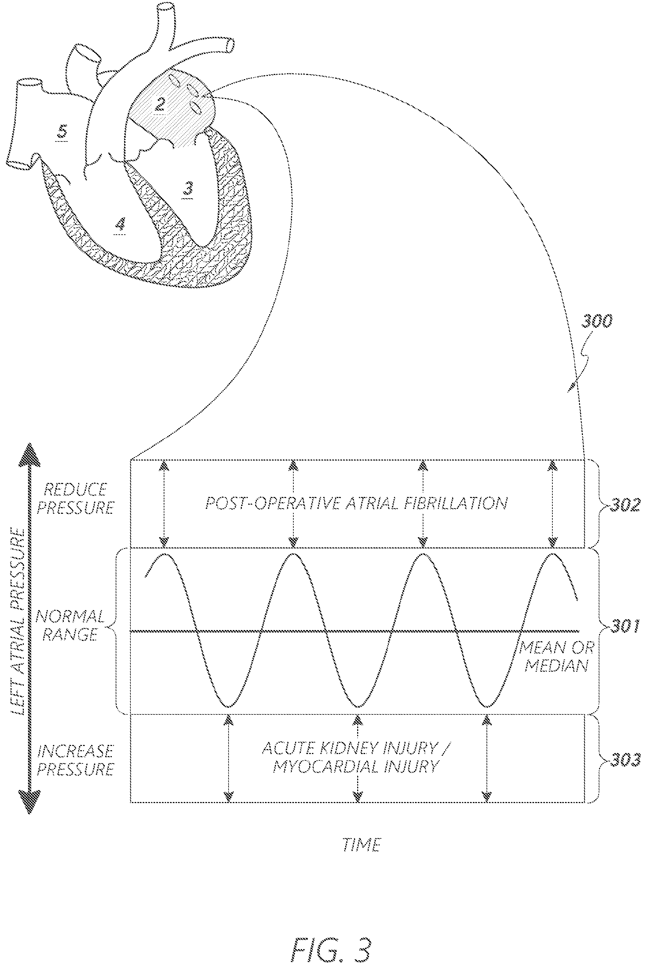

Figure 3 illustrates a graph 300 showing left atrial pressure ranges including a normal range 301 of left atrial pressure that is not generally associated with substantial risk of postoperative atrial fibrillation, acute kidney injury, myocardial injury, heart failure and/or other health conditions. Examples of the present disclosure provide systems, devices, and methods for determining whether a patient's left atrial pressure is within the normal range 301, above the normal range 303, or below the normal range 302 through the use of certain sensor implant devices. For detected left atrial pressure above the normal range, which may be correlated with an increased risk of heart failure, examples of the present disclosure as described in detail below can inform efforts to reduce the left atrial pressure until it is brought within the normal range 301. Furthermore, for detected left atrial pressure that is below the normal range 301, which may be correlated with increased risks of acute kidney injury, myocardial injury, and/or other health complications, examples of the present disclosure as described in detail below can serve to facilitate efforts to increase the left atrial pressure to bring the pressure level within the normal range 301.

### Implant Devices with Integrated Sensors

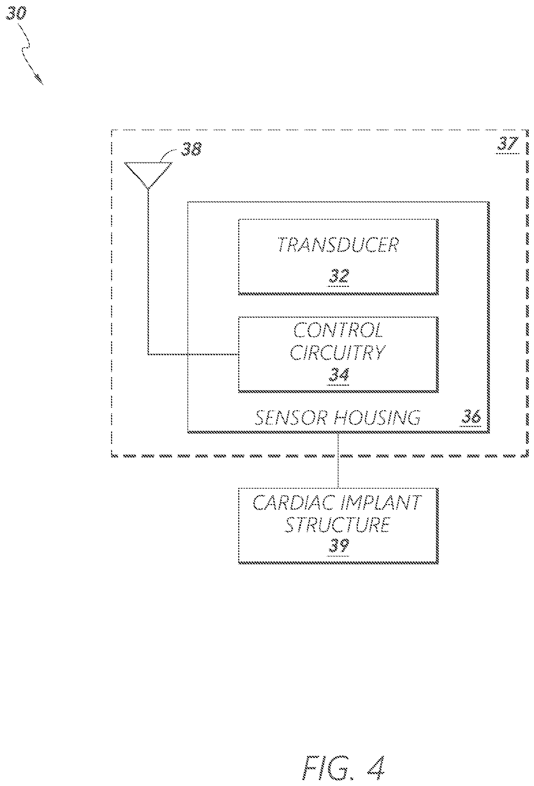

In some implementations, the present disclosure relates to sensors associated or integrated with cardiac shunts or other implant devices. Such integrated devices may be used to provide controlled and/or more effective therapies for treating and preventing heart failure and/or other health complications related to cardiac function. Figure 4 is a block diagram illustrating an implant device 30 comprising a shunt (or other type of implant) structure 39. In some examples, the shunt structure 39 is physically integrated with and/or connected to a sensor device 37. The sensor device 37 may be, for example, a pressure sensor, or other type of sensor. In some examples, the sensor 37 comprises a transducer 32, such as a pressure transducer, as well as certain control circuitry 34, which may be embodied in, for example, an application-specific integrated circuit (ASIC).

The control circuitry 34 may be configured to process signals received from the transducer 32 and/or communicate signals associated therewith wirelessly through biological tissue using the antenna 38. The term "control circuitry" is used herein according to its broad and ordinary meaning, and may refer to any collection of processors, processing circuitry, processing modules/units, chips, dies (e.g., semiconductor dies including come or more active and/or passive devices and/or connectivity circuitry), microprocessors, micro-controllers, digital signal processors, microcomputers, central processing units, field programmable gate arrays, programmable logic devices, state machines (e.g., hardware state machines), logic circuitry, analog circuitry, digital circuitry, and/or any device that manipulates signals (analog and/or digital) based on hard coding of the circuitry and/or operational instructions. Control circuitry referenced herein may further comprise one or more, storage devices, which may be embodied in a single memory device, a plurality of memory devices, and/or embedded circuitry of a device. Such data storage may comprise read-only memory, random access memory, volatile memory, non-volatile memory, static memory, dynamic memory, flash memory, cache memory, data storage registers, and/or any device that stores digital information. It should be noted that in examples in which control circuitry comprises a hardware and/or software state machine, analog circuitry, digital circuitry, and/or logic circuitry, data storage device(s)/register(s) storing any associated operational instructions may be embedded within, or external to, the circuitry comprising the state machine, analog circuitry, digital circuitry, and/or logic circuitry. The transducer(s) 32 and/or antenna(s) 38 can be considered part of the control circuitry 34.

The antenna 38 may comprise one or more coils or loops of conductive material, such as copper wire or the like. In some examples, at least a portion of the transducer 32, control circuitry 34, and/or the antenna 38 are at least partially disposed or contained within a sensor housing 36, which may comprise any type of material, and may advantageously be at least partially hermetically scaled. For example, the housing 36 may comprise glass or other rigid material in some examples, which may provide mechanical stability and/or protection for the components housed therein. In some examples, the housing 36 is at least partially flexible. For example, the housing may comprise polymer or other flexible structure/material, which may advantageously allow for folding, bending, or collapsing of the sensor 37 to allow for transportation thereof through a catheter or other introducing means.

The transducer 32 may comprise any type of sensor means or mechanism. For example, the transducer 32 may be a force-collector-type pressure sensor. In some examples, the transducer 32 comprises a diaphragm, piston, bourdon tube, bellows, or other strain- or deflection-measuring component(s) to measure strain or deflection applied over an area/surface thereof. The transducer 32 may be associated with the housing 36, such that at least a portion thereof is contained within or attached to the housing 36. With respect to sensor devices/components being "associated with" a stent or other implant structure, such terminology may refer to a sensor device or component being physically coupled, attached, or connected to, or integrated with, the implant structure.

In some examples, the transducer 32 comprises or is a component of a piezoresistive strain gauge, which may be configured to use a bonded or formed strain gauge to detect strain due to applied pressure, wherein resistance increases as pressure deforms the component/material. The transducer 32 may incorporate any type of material, including but not limited to silicon (e.g., monocrystalline), polysilicon thin film, bonded metal foil, thick film, silicon-on-sapphire, sputtered thin film, and/or the like.

In some examples, the transducer 32 comprises or is a component of a capacitive pressure sensor including a diaphragm and pressure cavity configured to form a variable capacitor to detect strain due to pressure applied to the diaphragm. The capacitance of the capacitive pressure sensor may generally decrease as pressure deforms the diaphragm. The diaphragm may comprise any material(s), including but not limited to metal, ceramic, silicon, and the like. In some examples, the transducer 32 comprises or is a component of an electromagnetic pressure sensor, which may be configured to measure the displacement of a diaphragm by means of changes in inductance, linear variable displacement transducer (LVDT) functionality, Hall Effect, or eddy current sensing. In some examples, the transducer 32 comprises or is a component of a piezoelectric strain sensor. For example, such a sensor may determine strain (e.g., pressure) on a sensing mechanism based on the piezoelectric effect in certain materials, such as quartz.

In some examples, the transducer 32 comprises or is a component of a strain gauge. For example, a strain gauge example may comprise a pressure sensitive element on or associated with an exposed surface of the transducer 32. In some examples, a metal strain gauge is adhered to a surface of the sensor, or a thin-film gauge may be applied on the sensor by sputtering or other technique. The measuring element or mechanism may comprise a diaphragm or metal foil. The transducer 32 may comprise any other type of sensor or pressure sensor, such as optical, potentiometric, resonant, thermal, ionization, or other types of strain or pressure sensors.

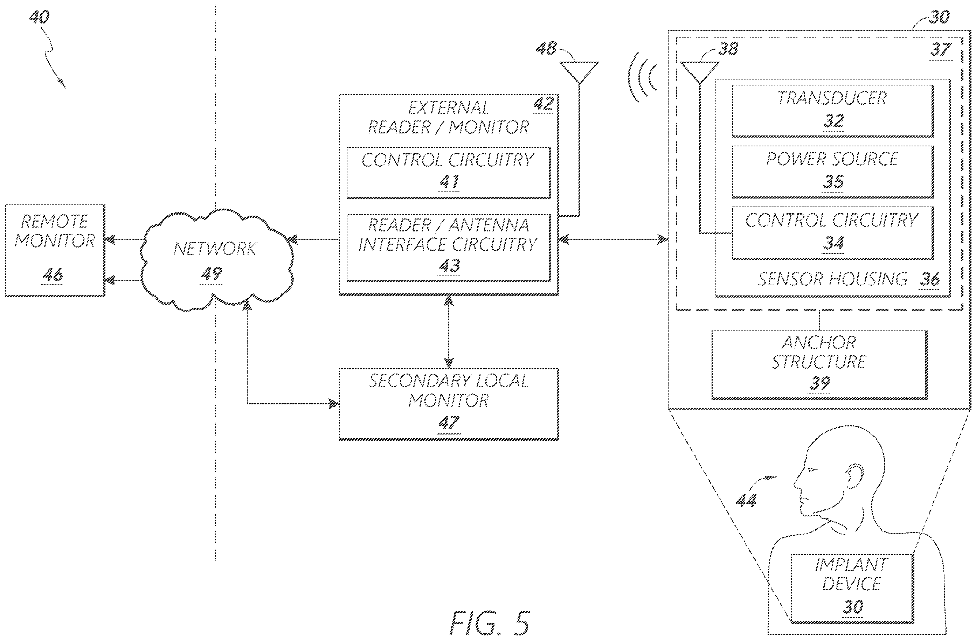

Figure 5 shows a system 40 for monitoring one or more physiological parameters (e.g., left atrial pressure and/or volume) in a patient 44 according to one or more examples. The patient 44 can have a medical implant device 30 implanted in, for example, the heart (not shown), or associated physiology, of the patient 44. For example, the implant device 30 can be implanted at least partially within the left atrium and/or coronary sinus of the patient's heart. The implant device 30 can include one or more sensor transducers 32, such as one or more microelectromechanical system (MEMS) devices (e.g., MEMS pressure sensors, or other type of sensor transducer).

In certain examples, the monitoring system 40 can comprise at least two subsystems, including an implantable internal subsystem or device 30 that includes the sensor transducer(s) 32, as well as control circuitry 34 comprising one or more microcontroller(s), discrete electronic component(s), and one or more power and/or data transmitter(s) 38 (e.g., antennae coil). The monitoring system 40 can further include an external (e.g., non-implantable) subsystem that includes an external reader 42 (e.g., coil), which may include a wireless transceiver that is electrically and/or communicatively coupled to certain control circuitry 41. In certain examples, both the internal 30 and external 42 subsystems include a corresponding coil antenna for wireless communication and/or power delivery through patient tissue disposed therebetween. The sensor implant device 30 can be any type of implant device. For example, in some examples, the implant device 30 comprises a pressure sensor integrated with another functional implant structure 39, such as a prosthetic shunt or stent device/structure.

Certain details of the implant device 30 are illustrated in the enlarged block 30 shown. The implant device 30 can comprise an implant/anchor structure 39 as described herein. For example, the implant/anchor structure 39 can include a percutaneously deliverable shunt device configured to be secured to and/or in a tissue wall to provide a flow path between two chambers and/or vessels of the heart, as described in detail throughout the present disclosure. Although certain components are illustrated in Figure 5 as part of the implant device 30, it should be understood that the sensor implant device 30 may only comprise a subset of the illustrated components/modules and can comprise additional components/modules not illustrated. The implant device may represent an example of the implant device shown in Figure 4, and vice versa. The implant device 30 can advantageously include one or more sensor transducers 32, which can be configured to provide a response indicative of one or more physiological parameters of the patient 44, such as atrial pressure. Although pressure transducers are described, the sensor transducer(s) 32 can comprise any suitable or desirable types of sensor transducer(s) for providing signals relating to physiological parameters or conditions associated with the implant device 30 and/or patient 44.

The sensor transducer(s) 32 can comprise one or more MEMS sensors, optical sensors, piezoelectric sensors, electromagnetic sensors, strain sensors/gauges, accelerometers, gyroscopes, diaphragm-based sensors, and/or other types of sensors, which can be positioned in the patient 44 to sense one or more parameters relevant to the health of the patient. The transducer 32 may be a force-collector-type pressure sensor. In some examples, the transducer 32 comprises a diaphragm, piston, bourdon tube, bellows, or other strain- or deflection-measuring component(s) to measure strain or deflection applied over an area/surface thereof. The transducer 32 may be associated with the sensor housing 36, such that at least a portion thereof is contained within, or attached to, the housing 36.

In some examples, the transducer 32 comprises or is a component of a strain gauge, which may be configured to use a bonded or formed strain gauge to detect strain due to applied pressure. For example, the transducer 32 may comprise or be a component of a piezoresistive strain gauge, wherein resistance increases as pressure deforms the component/material of the strain gauge. The transducer 32 may incorporate any type of material, including but not limited to silicone, polymer, silicon (e.g., monocrystalline), polysilicon thin film, bonded metal foil, thick film, silicon-on-sapphire, sputtered thin film, and/or the like. In some examples, a metal strain gauge is adhered to the sensor surface, or a thin-film gauge may be applied on the sensor by sputtering or other technique. The measuring element or mechanism may comprise a diaphragm or metal foil. The transducer 32 may comprise any other type of sensor or pressure sensor, such as optical, potentiometric, resonant, thermal, ionization, or other types of strain or pressure sensors.

In some examples, the transducer 32 comprises or is a component of a capacitive pressure sensor including a diaphragm and pressure cavity configured to form a variable capacitor to detect strain due to pressure applied to the diaphragm. The capacitance of the capacitive pressure sensor may generally decrease as pressure deforms the diaphragm. The diaphragm may comprise any material(s), including but not limited to metal, ceramic, silicone, silicon or other semiconductor, and the like. In some examples, the transducer 32 comprises or is a component of an electromagnetic pressure sensor, which may be configured to measures the displacement of a diaphragm by means of changes in inductance, linear variable displacement transducer (LVDT) functionality, Hall Effect, or eddy current sensing. In some examples, the transducer 32 comprises or is a component of a piezoelectric strain sensor. For example, such a sensor may determine strain (e.g., pressure) on a sensing mechanism based on the piezoelectric effect in certain materials, such as quartz.

In some examples, the transducer(s) 32 is/are electrically and/or communicatively coupled to the control circuitry 34, which may comprise one or more application-specific integrated circuit (ASIC) microcontrollers or chips. The control circuitry 34 can further include one or more discrete electronic components, such as tuning capacitors, resistors, diodes, inductors, or the like.

In certain examples, the sensor transducer(s) 32 can be configured to generate electrical signals that can be wirelessly transmitted to a device outside the patient's body, such as the illustrated local external monitor system 42. In order to perform such wireless data transmission, the implant device 30 can include radio frequency (RF) (or other frequency band) transmission circuitry, such as signal processing circuitry and an antenna 38. The antenna 38 can comprise an antenna coil implanted within the patient. The control circuitry 34 may comprise any type of transceiver circuitry configured to transmit an electromagnetic signal, wherein the signal can be radiated by the antenna 38, which may comprise one or more conductive wires, coils, plates, or the like. The control circuitry 34 of the implant device 30 can comprise, for example, one or more chips or dies configured to perform some amount of processing on signals generated and/or transmitted using the device 30. However, due to size, cost, and/or other constraints, the implant device 30 may not include independent processing capability in some examples.

The wireless signals generated by the implant device 30 can be received by the local external monitor device or subsystem 42, which can include a reader/antenna-interface circuitry module 43 configured to receive the wireless signal transmissions from the implant device 30, which is disposed at least partially within the patient 44. For example, the module 43 may include transceiver device(s)/circuitry.

The external local monitor 42 can receive the wireless signal transmissions from the implant device 30 and/or provide wireless power to the implant device 30 using an external antenna 48, such as a wand device. The reader/antenna-interface circuitry 43 can include radiofrequency (RF) (or other frequency band) front-end circuitry configured to receive and amplify the signals from the implant device 30, wherein such circuitry can include one or more filters (e.g., band-pass filters), amplifiers (e.g., low-noise amplifiers), analog-to-digital converters (ADC) and/or digital control interface circuitry, phase-locked loop (PLL) circuitry, signal mixers, or the like. The reader/antenna-interface circuitry 43 can further be configured to transmit signals over a network 49 to a remote monitor subsystem or device 46. The RF circuitry of the reader/antenna-interface circuitry 43 can further include one or more of digital-to-analog converter (DAC) circuitry, power amplifiers, low-pass filters, antenna switch modules, antennas, or the like for treatment/processing of transmitted signals over the network 49 and/or for receiving signals from the implant device 30. In certain examples, the local monitor 42 includes control circuitry 41 for performing processing of the signals received from the implant device 30. The local monitor 42 can be configured to communicate with the network 49 according to a known network protocol, such as Ethernet, Wi-Fi, or the like. In certain examples, the local monitor 42 comprises a smartphone, laptop computer, or other mobile computing device, or any other type of computing device.

In certain examples, the implant device 30 includes some amount of volatile and/or non-volatile data storage. For example, such data storage can comprise solid-state memory utilizing an array of floating-gate transistors, or the like. The control circuitry 34 may utilize data storage for storing sensed data collected over a period of time, wherein the stored data can be transmitted periodically to the local monitor 42 or other external subsystem. In certain examples, the implant device 30 does not include any data storage. The control circuitry 34 may be configured to facilitate wireless transmission of data generated by the sensor transducer(s) 32, or other data associated therewith. The control circuitry 34 may further be configured to receive input from one or more external subsystems, such as from the local monitor 42, or from a remote monitor 46 over, for example, the network 49. For example, the implant device 30 may be configured to receive signals that at least partially control the operation of the implant device 30, such as by activating/deactivating one or more components or sensors, or otherwise affecting operation or performance of the implant device 30.

The one or more components of the implant device 30 can be powered by one or more power sources 35. Due to size, cost and/or electrical complexity concerns, it may be desirable for the power source 35 to be relatively minimalistic in nature. For example, high-power driving voltages and/or currents in the implant device 30 may adversely affect or interfere with operation of the heart or other body part associated with the implant device. In certain examples, the power source 35 is at least partially passive in nature, such that power can be received from an external source wirelessly by passive circuitry of the implant device 30, such as through the use of short-range, or near-field wireless power transmission, or other electromagnetic coupling mechanism. For example, the local monitor 42 may serve as an initiator that actively generates an RF field that can provide power to the implant device 30, thereby allowing the power circuitry of the implant device to take a relatively simple form factor. In certain examples, the power source 35 can be configured to harvest energy from environmental sources, such as fluid flow, motion, or the like. Additionally or alternatively, the power source 35 can comprise a battery, which can advantageously be configured to provide enough power as needed over the monitoring period (e.g., 3, 5, 10, 20, 30, 40, or 90 days, or other period of time).

In some examples, the local monitor device 42 can serve as an intermediate communication device between the implant device 30 and the remote monitor 46. The local monitor device 42 can be a dedicated external unit designed to communicate with the implant device 30. For example, the local monitor device 42 can be a wearable communication device, or other device that can be readily disposed in proximity to the patient 44 and implant device 30. The local monitor device 42 can be configured to continuously, periodically, or sporadically interrogate the implant device 30 in order to extract or request sensor-based information therefrom. In certain examples, the local monitor 42 comprises a user interface, wherein a user can utilize the interface to view sensor data, request sensor data, or otherwise interact with the local monitor system 42 and/or implant device 30.

The system 40 can include a secondary local monitor 47, which can be, for example, a desktop computer or other computing device configured to provide a monitoring station or interface for viewing and/or interacting with the monitored cardiac pressure data. In an example, the local monitor 42 can be a wearable device or other device or system configured to be disposed in close physical proximity to the patient and/or implant device 30, wherein the local monitor 42 is primarily designed to receive/transmit signals to and/or from the implant device 30 and provide such signals to the secondary local monitor 47 for viewing, processing, and/or manipulation thereof. The external local monitor system 42 can be configured to receive and/or process certain metadata from or associated with the implant device 30, such as device ID or the like, which can also be provided over the data coupling from the implant device 30.

The remote monitor subsystem 46 can be any type of computing device or collection of computing devices configured to receive, process and/or present monitor data received over the network 49 from the local monitor device 42, secondary local monitor 47, and/or implant device 30. For example, the remote monitor subsystem 46 can advantageously be operated and/or controlled by a healthcare entity, such as a hospital, doctor, or other care entity associated with the patient 44. Although certain examples disclosed herein describe communication with the remote monitor subsystem 46 from the implant device indirectly through the local monitor device 42, in certain examples, the implant device 30 can comprise a transmitter capable of communicating over the network 49 with the remote monitor subsystem 46 without the necessity of relaying information through the local monitor device 42.

In some examples, at least a portion of the transducer 32, control circuitry 34, power source 35 and/or the antenna 38 are at least partially disposed or contained within the sensor housing 36, which may comprise any type of material, and may advantageously be at least partially hermetically sealed. For example, the housing 36 may comprise glass or other rigid material in some examples, which may provide mechanical stability and/or protection for the components housed therein. In some examples, the housing 36 is at least partially flexible. For example, the housing may comprise polymer or other flexible structure/material, which may advantageously allow for folding, bending, or collapsing of the sensor 37 to allow for transportation thereof through a catheter or other percutaneous introducing means.

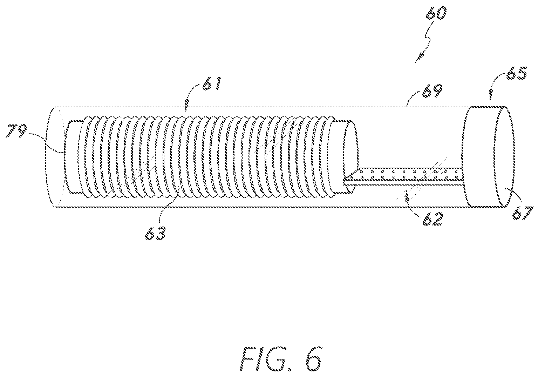

As referenced above, shunt and other implant devices/structures may be integrated with sensor, antenna/transceiver, and/or other components to facilitate in vivo monitoring of pressure and/or other physiological parameter(s). Sensor devices in accordance with examples of the present disclosure may be integrated with cardiac shunt structures/devices or other implant devices using any suitable or desirable attachment or integration mechanism or configuration. Figure 6 illustrates an example sensor assembly/device 60 that can be a component of a sensor implant device. The sensor device 60 may be configured to provide sensor readings relating to one or more physiological parameters associated with a target implantation site.

The sensor device 60 may be configured for attachment to implant devices. For example, a coil form including one or more wires or other material or structure shaped into one or more winds of coil forming a fluid conduit/barrel portion and axial end flanges may be used to attach the sensor device 60 to one or more implants. A shunt structure may be integrated with pressure sensor functionality in accordance with certain examples disclosed herein. The shunt structure may be configured to hold the sensor device 60.

The sensor device 60 may advantageously be disposed, positioned, secured, oriented, and/or otherwise situated in a configuration in which a sensor transducer component 65 thereof is disposed within a channel area of a shunt structure. The term "channel area" is used herein according to its broad and ordinary meaning and may refer to a three-dimensional space defined by a radial boundary of a fluid conduit and extending axially from the fluid conduit.

In some examples, the sensor assembly 61 includes a sensor component 65 and an antenna component 69. The sensor component 65 may comprise any type of sensor device as described in detail above. In some examples, the sensor 65 may be attached to or integrated with an arm member of a shunt structure.

The sensor 65 includes a sensor element 67, such as a pressure sensor transducer. As described herein, the sensor assembly 61 may be configured to implement wireless data and/or power transmission. The sensor assembly 61 may include an antenna component 69 for such purpose. The antenna 69 may be contained at least partially within an antenna housing 79, which may further have disposed therein certain control circuitry configured to facilitate wireless data and/or power communication functionality. In some examples, the antenna component 69 comprises one or more conductive coils 62, which may facilitate inductive powering and/or data transmission. In examples comprising conductive coil(s), such coil(s) may be wrapped/disposed at least partially around a magnetic (e.g., ferrite, iron) core 63.

The antenna component 69 may be attached to, integrated with, or otherwise associated with an arm/anchor feature of a shunt structure.

The sensor assembly 61 may advantageously be biocompatible. For example, the sensor 65 and antenna 69 may comprise biocompatible housings, such as a housing comprising glass or other biocompatible material. However, at least a portion of the sensor element 67, such as a diaphragm or other component, may be exposed to the external environment in some examples in order to allow for pressure readings, or other parameter sensing, to be implemented. With respect to the antenna housing 79, the housing 79 may comprise an at least partially rigid cylindrical or tube-like form, such as a glass cylinder form. In some examples, the sensor 65/67 component is approximately 3 mm or less in diameter. The antenna 69 may be approximately 20 mm or less in length.

The sensor assembly 61 may be configured to communicate with an external system when implanted in a heart or other area of a patient's body. For example, the antenna 69 may receive power wirelessly from the external system and/or communicate sensed data or waveforms to and/or from the external system. The sensor assembly 61 may be attached to, or integrated with, a shunt structure in any suitable or desirable way. For example, in some implementations, the sensor 65 and/or antenna 69 may be attached or integrated with the shunt structure using mechanical attachment means. In some examples, the sensor 65 and/or antenna 69 may be contained in a pouch or other receptacle that is attached to a shunt structure.

The sensor element 67 may comprise a pressure transducer. For example, the pressure transducer may be a microelectromechanical system (MEMS) transducer comprising a semiconductor diaphragm component. In some examples, the transducer may include an at least partially flexible or compressible diaphragm component, which may be made from silicone or other flexible material. The diaphragm component may be configured to be flexed or compressed in response to changes in environmental pressure.

### Sensor Implant Devices

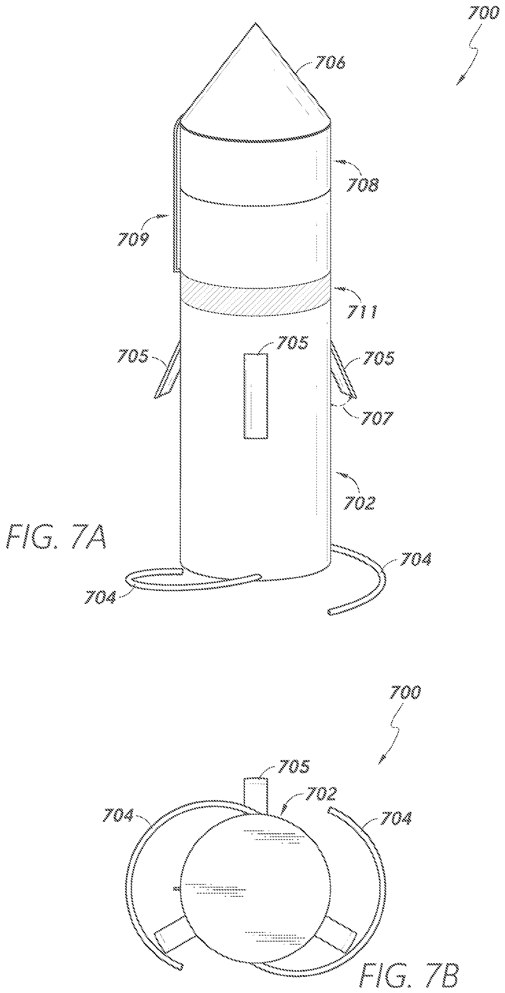

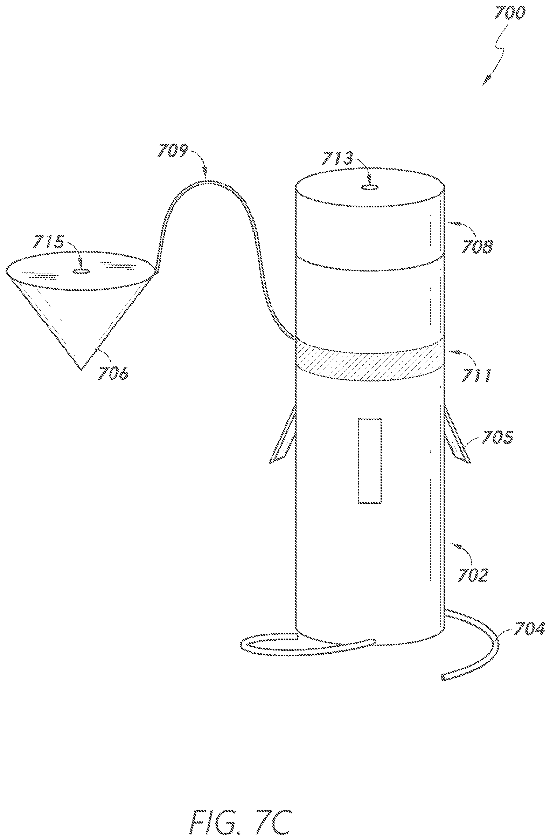

Figures 7A-7C illustrate a sensor implant device 700 in accordance with one or more embodiments of the presently claimed invention. Figure 7A provides a side view of the device 700 in a compressed and/or delivery form, Figure 7B provides a bottom view of the device 700 in the compressed and/or delivery form, and Figure 7C provides a side view of the device 700 in an expanded and/or anchored form. The sensor implant device 700 comprises a sensor body 702 coupled, attached, and/or otherwise releasably and/or permanently secured to one or more anchoring features. The term "anchoring feature" is used herein in accordance with its plain and ordinary meaning and may refer to any means for anchoring, which can include one or more clips, coils, puncture coils, hooks, arms, cords, spikes, needles, and/or other features configured for anchoring and/or attachment at one or more areas of tissue within a heart.

In some examples, the device 700 may comprise multiple types of anchoring features, which can include one or more end anchors 704 and/or one or more side anchors 705. An end anchor 704 can have a form of a coiled and/or curved wire and/or cord. In some examples, the end anchor 704 can have a generally helical form and/or may extend laterally and/or longitudinally away from the sensor body 702. An end anchor 704 may be configured to curve about a longitudinal axis of the sensor body 702. In some examples, the device 700 can comprise multiple end anchors 704, as shown in Figures 7A-7C. For example, the device 700 can comprise two end anchors 704. In some examples, the end anchors 704 may be configured to extend from generally opposite sides of the sensor body 702 to maximize coverage of the end anchors 704 around a circumference of the sensor body 702. In some examples, an end anchor 704 may have a generally semi-circular shape. For example, the one or more end anchors 704 may have semi-circular shapes that may be generally concentric with the sensor body 702 and/or with each other. The one or more end anchors 704 may be configured to increase a lateral profile (i.e., width) of the sensor device 700 and/or to press against and/or contact a tissue wall surface to prevent the sensor device 700 from extending further into and/or out of the tissue wall. In some examples, the one or more end anchors 704 may be configured to compress against and/or in line with the sensor body 702 during delivery of the device 700 (e.g., via a catheter).

The device 700 may additionally or alternatively comprise one or more side anchors 705 configured to extend from a midsection of the sensor body 702. The side anchors 705 may be configured to embed into and/or press against tissue within an opening through a tissue wall. For example, the one or more side anchors 705 may comprise spikes and/or needles configured to pierce and/or puncture a tissue wall. In some examples, the one or more side anchors 705 may be configured to compress against the sensor body 702 during delivery of the device 700 (e.g., via a catheter) and/or may be configured to extend (e.g., naturally and/or by default) to the expanded form shown in Figures 7A-7C. The one or more side anchors 705 may be attached to the sensor body 702 at a first side and/or may be configured to swing freely (e.g., via a hinge attachment at the first side) away from the sensor body 702. In some examples, the one or more side anchors 705 may be configured to extend out to an acute angle 707 (e.g., approximately 45-degrees and/or less than 90-degrees) with respect to the sensor body 702. The one or more side anchors 705 may be configured to anchor to the tissue wall when the sensor device 700 is at least partially pulled back and/or retracted into the tissue wall. For example, the device 700 may be extended into a tissue wall while the one or more side anchors 705 remain compressed against the sensor body 702. After at least a portion of the sensor device 700 (e.g., a leading end 706 and/or distal portion 708) extends entirely through the tissue wall, the device 700 may be at least partially retracted to cause the side anchors 705 to extend away from the sensor body 702 and/or to embed into the tissue.

In some examples, the one or more side anchors 705 may be configured to extend in multiple directions around the sensor body 702 and/or may extend in generally opposite directions laterally (i.e., along a diameter of the sensor body 702) from the sensor body 702. The one or more side anchors 705 may extend linearly and/or non-linearly away from and/or along the sensor body 702. In some examples, the one or more side anchors 705 extend longitudinally (i.e., along a shaft of the sensor body 702) along the sensor body 702.

The sensor device 700 comprises at least one sensor component. In some examples, a sensor component may be situated at or near a distal portion 708 of the sensor body 702. The sensor component(s) may comprise any type of sensor device(s) as described in detail above. The sensor implant device 700 may be configured to position the sensor component(s) at a target location within a body, which can include a heart chamber (e.g., a left atrium), an opening (e.g., a left atrial appendage) into and/or from a heart chamber, and/or a blood flow pathway (e.g., a coronary sinus).

The device 700 comprises a detachable tip 706. While the detachable tip 706 is shown having a conical and/or pointed form, the detachable tip 706 may have any suitable shape and/or size. In some examples, the detachable tip 706 may be configured to provide a leading end and/or dilating end of the device 700 during delivery. For example, the detachable tip 706 may be situated at or near the distal portion 708 of the device 700 during delivery of the device 700 and/or may be configured to pierce, puncture, and/or dilate one or more blood vessels and/or tissue walls. The detachable tip 706 may comprise a generally flat base and/or side configured to contact and/or lay flatly and/or rest against a distal end of the sensor body 702.

As shown in Figure 7C, the detachable tip 706 is configured to disconnect from the sensor body 702 and is tethered to the sensor body 702 via a tether 709, which can include a wire, cord, and/or similar device. In some examples, the tether 709 may be at least partially composed of Nitinol and/or other shape memory alloys and/or may otherwise have shape memory features. For example, the tether 709 may be shape set in the form shown in Figure 7C such that the tether 709 may be configured to naturally pull the detachable tip 706 away from the sensor body 702. The detachable tip 706 may be configured to become detached from the sensor body 702 once the detachable tip 706 passes through and/or exits a tissue wall.

In some examples, the device 700 may comprise one or more markers 711 (e.g., radiopaque markers) configured to facilitate delivery and/or tracking of the device 700. A marker 711 may be situated along the sensor body 702.

The sensor body 702 may comprise an inner lumen 713 extending at least partially through the sensor body 702. The inner lumen 713 may be configured to receive and/or facilitate delivery of one or more guidewires. In some examples, the detachable tip 706 may comprise a lumen 715 configured to align with the inner lumen 713 of the sensor body 702.

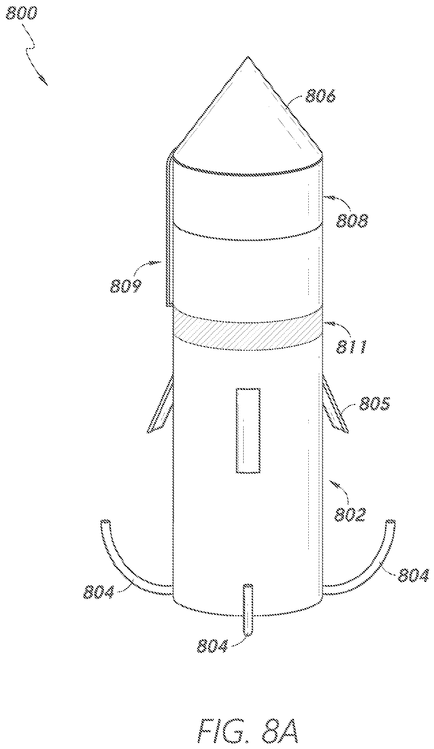

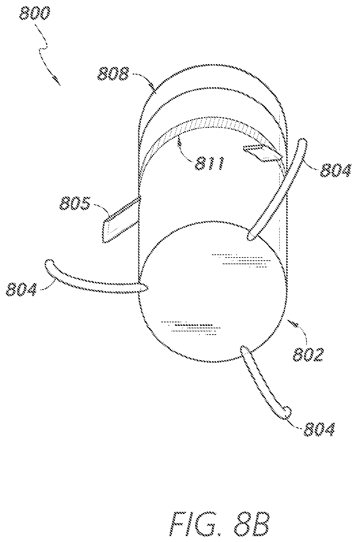

Figures 8A and 8B illustrate another example sensor implant device 800 in accordance with one or more embodiments of the presently claimed invention. Figure 8A provides a side view of the device 800 in a compressed and/or delivery form and Figure 8B provides a perspective view of the device 800 in the compressed and/or delivery form, and Figure 8C provides a side view of the device 800 in an expanded and/or anchored form. The sensor implant device 800 comprises a sensor body 802 coupled, attached, and/or otherwise releasably and/or permanently secured to one or more anchoring features. The term "anchoring feature" is used herein in accordance with its plain and ordinary meaning and may refer to any means for anchoring, which can include one or more clips, coils, puncture coils, hooks, arms, cords, spikes, needles, and/or other features configured for anchoring and/or attachment at one or more areas of tissue within a heart.

In some examples, the device 800 may comprise multiple types of anchoring features, which can include one or more end anchors 804 and/or one or more side anchors 805. An end anchor 804 can have a form of a curved wire and/or a hook. In some examples, one or more end anchors 804 can have a generally curved form and/or may extend laterally away from the sensor body 802 and/or longitudinally along the sensor body 802. In some examples, the device 800 can comprise multiple end anchors 804, as shown in Figures 8A and 8B. For example, the device 800 can comprise three end anchors 804. In some examples, the end anchors 804 may be configured to extend from generally opposite sides of the sensor body 802 to maximize coverage of the end anchors 804 around a circumference of the sensor body 802. In some examples, an end anchor 804 may have a generally semi-circular shape. The one or more end anchors 804 may be configured to increase a lateral profile (i.e., width) of the sensor device 800 and/or to press against and/or contact a tissue wall surface to prevent the sensor device 800 from extending further into and/or out of the tissue wall. In some examples, the one or more end anchors 804 may be configured to compress against and/or in line with the sensor body 802 during delivery of the device 800 (e.g., via a catheter).

The device 800 may additionally or alternatively comprise one or more side anchors 805 configured to extend from a midsection of the sensor body 802. The side anchors 805 may be configured to embed into and/or press against tissue within an opening through a tissue wall. For example, the one or more side anchors 805 may comprise spikes and/or needles configured to pierce and/or puncture a tissue wall. In some examples, the one or more side anchors 805 may be configured to compress against the sensor body 802 during delivery of the device 800 (e.g., via a catheter) and/or may be configured to extend (e.g., naturally and/or by default) to the expanded form shown in Figures 8A and 8B. The one or more side anchors 805 may be attached to the sensor body 802 at a first side and/or may be configured to swing freely (e.g., via a hinge attachment at the first side) away from the sensor body 802. In some examples, the one or more side anchors 805 may be configured to extend out to an acute angle (e.g., approximately 45-degrees and/or less than 90-degrees) with respect to the sensor body 802. The one or more side anchors 805 may be configured to anchor to the tissue wall when the sensor device 800 is at least partially pulled back and/or retracted into the tissue wall. For example, the device 800 may be extended into a tissue wall while the one or more side anchors 805 remain compressed against the sensor body 802. After at least a portion of the sensor device 800 (e.g., a leading end 806 and/or distal portion 808) extends entirely through the tissue wall, the device 800 may be at least partially retracted to cause the side anchors 805 to extend away from the sensor body 802 and/or to embed into the tissue.

In some examples, the one or more side anchors 805 may be configured to extend in multiple directions around the sensor body 802 and/or may extend in generally opposite directions laterally (i.e., along a diameter of the sensor body 802) from the sensor body 802. The one or more side anchors 805 may extend linearly and/or non-linearly away from and/or along the sensor body 802. In some examples, the one or more side anchors 805 extend longitudinally (i.e., along a shaft of the sensor body 802) along the sensor body 802.

The sensor device 800 comprises at least one sensor component. In some examples, a sensor component may be situated at or near a distal portion 808 of the sensor body 802. The sensor component(s) may comprise any type of sensor device(s) as described in detail above. The sensor implant device 800 may be configured to position the sensor component(s) at a target location within a body, which can include a heart chamber (e.g., a left atrium), an opening (e.g., a left atrial appendage) into and/or from a heart chamber, and/or a blood flow pathway (e.g., a coronary sinus).

The device 800 comprises a detachable tip 806. While the detachable tip 806 is shown having a conical and/or pointed form, the detachable tip 806 may have any suitable shape and/or size. In some examples, the detachable tip 806 may be configured to provide a leading end and/or dilating end of the device 800 during delivery. For example, the detachable tip 806 may be situated at or near the distal portion 808 of the device 800 during delivery of the device 800 and/or may be configured to pierce, puncture, and/or dilate one or more blood vessels and/or tissue walls.

The detachable tip 806 is configured to disconnect from the sensor body 802 andis tethered to the sensor body 802 via a tether 809, which can include a wire, cord, and/or similar device. In some examples, the tether 809 may be at least partially composed of Nitinol and/or other shape memory alloys and/or may otherwise have shape memory features. The tether 809 is shape set such that the tether 809 may be configured to naturally pull the detachable tip 806 away from the sensor body 802. The detachable tip 806 may be configured to become detached from the sensor body 802 once the detachable tip 806 passes through and/or exits a tissue wall.

In some examples, the device 800 may comprise one or more markers 811 (e.g., radiopaque markers) configured to facilitate delivery and/or tracking of the device 800. A marker 811 may be situated along the sensor body 802. In some examples, the marker 811 can comprise a ring-shaped mechanism fitted around a circumferential area of the sensor body 802.

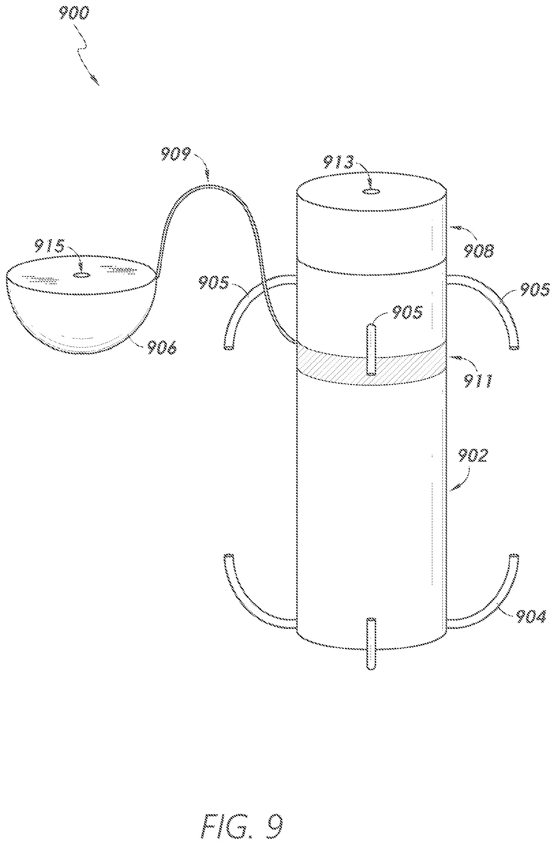

Figure 9 provides a perspective view of another example sensor implant device 900 in accordance with one or more embodiments of the presently claimed invention. The sensor implant device 800 comprises a sensor body 802 coupled, attached, and/or otherwise releasably and/or permanently secured to one or more anchoring features. The term "anchoring feature" is used herein in accordance with its plain and ordinary meaning and may refer to any means for anchoring, which can include one or more clips, coils, puncture coils, hooks, arms, cords, spikes, needles, and/or other features configured for anchoring and/or attachment at one or more areas of tissue within a heart.

In some examples, the device 900 may comprise multiple types of anchoring features, which can include one or more proximal end anchors 904 and/or one or more distal end anchors 905. While the device 900 is not shown comprising side anchors as illustrated in Figures 7 and 8, the device 900 may additionally or alternatively comprise side anchors.

A proximal end anchor 904 and/or distal end anchor 905 can have a form of a curved wire and/or a hook. In some examples, the one or more proximal end anchors 904 and/or distal end anchors 905 can have generally curved forms and/or may extend laterally away from the sensor body 902 and/or longitudinally along the sensor body 902. The proximal end anchors 904 may extend towards the distal end anchors 905 and/or the distal end anchors 905 may extend towards the proximal end anchors 904. In some examples, the device 900 can comprise multiple proximal end anchors 904 and/or distal end anchors 905, as shown in Figure 9. For example, the device 900 can comprise three or four proximal end anchors 904 and/or three or four distal end anchors 905. In some examples, the proximal end anchors 904 and/or distal end anchors 905 may be configured to extend from generally opposite sides of the sensor body 902 to maximize coverage of the proximal end anchors 904 and/or distal end anchors 905 around a circumference of the sensor body 902. In some examples, a proximal end anchor 904 and/or distal end anchor 905 may have a generally semi-circular shape. The one or more proximal end anchors 904 and/or distal end anchors 905 may be configured to increase a lateral profile (i.e., width) of the sensor device 900 and/or to press against and/or contact a tissue wall surface to prevent the sensor device 900 from extending further into and/or out of the tissue wall. In some examples, the one or more proximal end anchors 904 and/or distal end anchors 905 may be configured to compress against and/or in line with the sensor body 902 during delivery of the device 900 (e.g., via a catheter).

The device 900 comprises a detachable tip 906. While the detachable tip 906 is shown having a domed, rounded, and/or half-spherical form, the detachable tip 906 may have any suitable shape and/or size. In some examples, the detachable tip 906 may be configured to provide a leading end and/or dilating end of the device 900 during delivery. For example, the detachable tip 906 may be situated at or near the distal portion 908 of the device 900 during delivery of the device 900 and/or may be configured to pierce, puncture, and/or dilate one or more blood vessels and/or tissue walls. In some examples, a rounded end of the detachable tip 906 may be configured to prevent damage to surrounding tissue.

The device 900 comprises a detachable tip 906 configured to disconnect and/or detach from the sensor body 902 and is tethered to the sensor body 902 via a tether 909, which can include a wire, cord, and/or similar device. In some examples, the tether 909 may be at least partially composed of Nitinol and/or other shape memory alloys and/or may otherwise have shape memory features. For example, the tether 909 may be shape set in the form shown in Figure 9 such that the tether 909 may be configured to naturally pull the detachable tip 906 away from the sensor body 902. The detachable tip 906 may be configured to become detached from the sensor body 902 once the detachable tip 906 passes through and/or exits a tissue wall.

In some examples, the device 900 may comprise one or more markers 911 (e.g., radiopaque markers) configured to facilitate delivery and/or tracking of the device 900. A marker 911 may be situated along the sensor body 902.

The sensor body 902 may comprise an inner lumen 913 extending at least partially through the sensor body 902. The inner lumen 913 may be configured to receive and/or facilitate delivery of one or more guidewires. In some examples, the detachable tip 906 may comprise a lumen 915 configured to align with the inner lumen 913 of the sensor body 902.

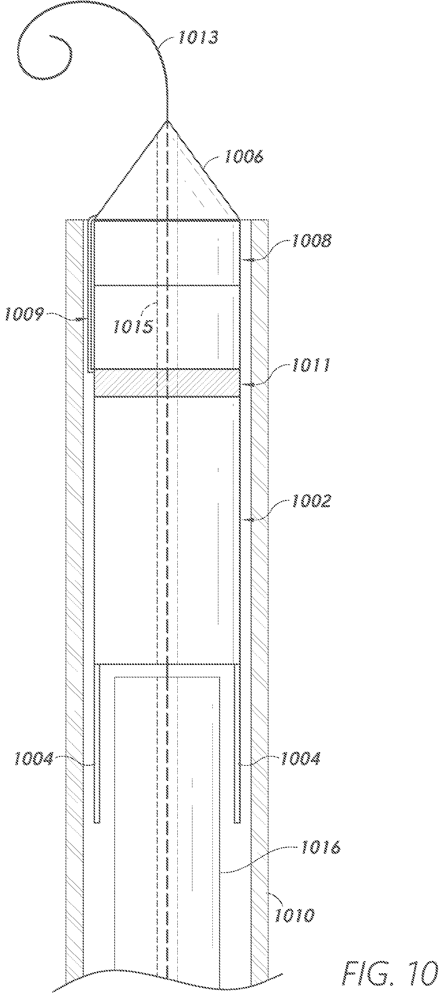

Figure 10 illustrates a delivery system for delivering a sensor implant device of the presently claimed invention in accordance with one or more examples. The sensor implant device comprises a sensor body 1002 coupled, attached, and/or otherwise releasably and/or permanently secured to one or more anchors 1004 (e.g., proximal end anchors).

While the device is not shown with side anchors for illustrative purposes, the device may comprise one or more side anchors extending from the sensor body 1002. An end anchor 1004 can have a form of a coiled and/or curved wire and/or cord. In some examples, the end anchor 1004 can have a generally helical form and/or may extend laterally and/or longitudinally away from the sensor body 1002. An end anchor 1004 may be configured to curve about a longitudinal axis of the sensor body 1002. In some examples, the device can comprise multiple end anchors 1004, as shown in Figure 10. For example, the device can comprise two end anchors 1004. In some examples, the end anchors 1004 may be configured to extend from generally opposite sides of the sensor body 1002 to maximize coverage of the end anchors 1004 around a circumference of the sensor body 1002. In some examples, an end anchor 1004 may have a generally semi-circular shape. The one or more end anchors 1004 may be configured to increase a lateral profile (i.e., width) of the sensor device and/or to press against and/or contact a tissue wall surface to prevent the sensor device from extending further into and/or out of the tissue wall.

The device is shown at least partially within a catheter 1010 and/or shaft. In some examples, the one or more end anchors 1004 may be configured to compress against and/or in line with the sensor body 1002 during delivery of the device (e.g., via the catheter 1010). For example, the catheter 1010 may be configured to at least partially enclose the device and/or prevent maximum expansion of the one or more anchors 1004.

The device may additionally or alternatively comprise one or more side anchors configured to extend from a midsection of the sensor body 1002. The side anchors may be configured to embed into and/or press against tissue within an opening through a tissue wall. For example, the one or more side anchors may comprise spikes and/or needles configured to pierce and/or puncture a tissue wall. In some examples, the one or more side anchors may be configured to compress against the sensor body 1002 during delivery of the device (e.g., via a catheter) and/or may be configured to extend (e.g., naturally) to the expanded form shown in Figure 10. The one or more side anchors may be attached to the sensor body 1002 at a first side and/or may be configured to swing freely (e.g., via a hinge attachment at the first side) away from the sensor body 1002. In some examples, the one or more side anchors may be configured to extend out to an acute angle (e.g., approximately 45-degrees and/or less than 90-degrees) with respect to the sensor body 1002. The one or more side anchors may be configured to anchor to the tissue wall when the sensor device is at least partially pulled back and/or retracted into the tissue wall. For example, the device may be extended into a tissue wall while the one or more side anchors remain compressed against the sensor body 1002. After at least a portion of the sensor device (e.g., a leading end 1006 and/or distal portion 1008) extends entirely through the tissue wall, the device may be at least partially retracted to cause the side anchors to extend away from the sensor body 1002 and/or to embed into the tissue.

In some examples, the one or more side anchors may be configured to extend in multiple directions around the sensor body 1002 and/or may extend in generally opposite directions laterally (i.e., along a diameter of the sensor body 1002) from the sensor body 1002. The one or more side anchors may extend linearly and/or non-linearly away from and/or along the sensor body 1002. In some examples, the one or more side anchors extend longitudinally (i.e., along a shaft of the sensor body 1002) along the sensor body 1002.

The sensor device comprises at least one sensor component. In some examples, a sensor component may be situated at or near a distal portion 1008 of the sensor body 1002. The sensor component(s) may comprise any type of sensor device(s) as described in detail above. The sensor implant device may be configured to position the sensor component(s) at a target location within a body, which can include a heart chamber (e.g., a left atrium), an opening (e.g., a left atrial appendage) into and/or from a heart chamber, and/or a blood flow pathway (e.g., a coronary sinus).

The device comprises a detachable tip 1006. While the detachable tip 1006 is shown having a conical and/or pointed form, the detachable tip 1006 may have any suitable shape and/or size. In some examples, the detachable tip 1006 may be configured to provide a leading end and/or dilating end of the device during delivery. For example, the detachable tip 1006 may be situated at or near the distal portion 1008 of the device during delivery of the device and/or may be configured to pierce, puncture, and/or dilate one or more blood vessels and/or tissue walls. The detachable tip 1006 may be configured to extend at least partially beyond a distal end of the catheter 1010 during delivery into and/or through a patient's body and/or to a tissue wall, as shown in Figure 10.

The detachable tip 1006 is configured to disconnect from the sensor body 1002 and is tethered to the sensor body 1002 via a tether 1009, which can include a wire, cord, and/or similar device. In some examples, the tether 1009 may be at least partially composed of Nitinol and/or other shape memory alloys and/or may otherwise have shape memory features. The tether 1009 is shape set such that the tether 1009 is configured to naturally pull the detachable tip 1006 away from the sensor body 1002. The detachable tip 1006 may be configured to become detached from the sensor body 1002 once the detachable tip 1006 passes through and/or exits a tissue wall.

In some examples, the device may comprise one or more markers 1011 (e.g., radiopaque markers) configured to facilitate delivery and/or tracking of the device. A marker 1011 may be situated along the sensor body 1002.

The sensor body 1002 may comprise an inner lumen 1015 extending at least partially through the sensor body 1002. The inner lumen 1015 may be configured to receive and/or facilitate delivery of one or more guidewires 1013. In some examples, the detachable tip 1006 may comprise a lumen 1015 configured to align with the inner lumen 1015 of the sensor body 1002.

In some examples, a guidewire 1013 may be configured to hold the detachable tip 1006 in place and/or in contact with the sensor body 1002. The sensor body 1002 and/or detachable tip 1006 may additionally or alternatively comprise various features (e.g., hooks, clasps, pegs, notches, grips, fasteners, grooves, glue, magnets, etc.) configured to maintain contact between the sensor body 1002 and the detachable tip 1006 during the delivery process.

In some examples, a pusher 1016 and/or shaft may be used to press the sensor device out of the catheter 1010 and/or beyond a distal end of the catheter 1010. The pusher 1016 may be configured to press against a proximal end of the sensor body 1002, as shown in Figure 10.

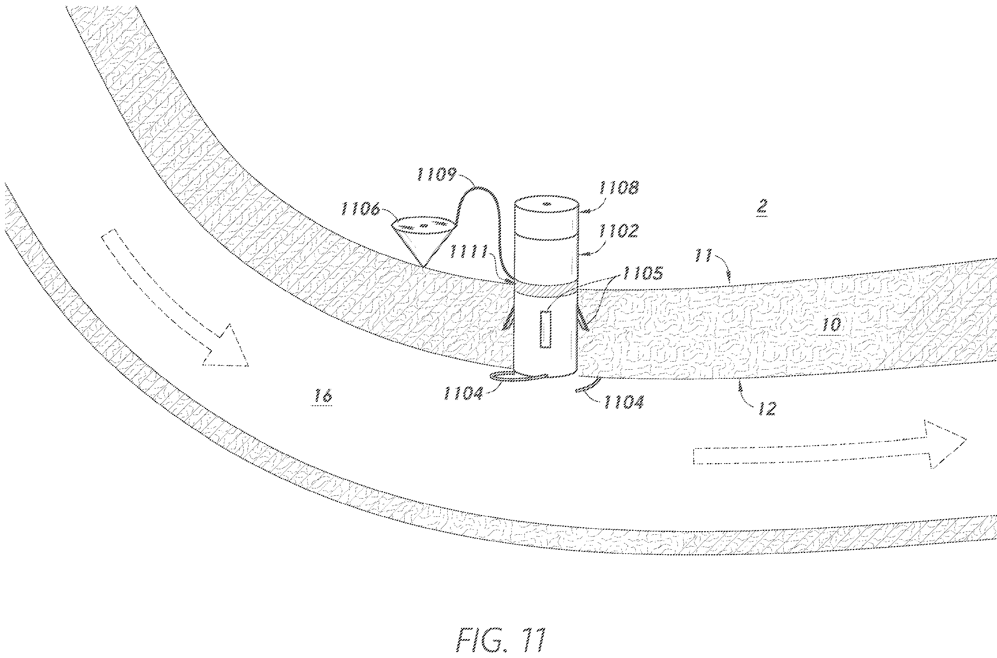

Figure 11 illustrates an example sensor device in accordance with embodiments of the presently claimed invention embedded at least partially within a tissue wall 10 (e.g., a right atrium 2 wall) in accordance with one or more examples. The sensor device can comprise a sensor body 1102, one or more proximal end anchors 1104, one or more side anchors 1105, a detachable tip 1106, and/or a distal portion 1108. The sensor device comprises one or more sensor components, which can be housed for example at the distal portion 1108.

The proximal end anchors 1104 may be configured to anchor the sensor device to a proximal surface 12 of the tissue wall 10. For example, the one or more proximal end anchors 1104 can extend from a proximal end of the sensor body 1102 and/or may increase a width and/or lateral profile of the sensor device at the proximal end. In some examples, the proximal end anchors 1104 may be configured to prevent the sensor body 1102 from becoming dislodged from the tissue wall 10 and/or from navigating upward into the right atrium 2.

The side anchors 1105 may extend from a midsection of the sensor body 1102 and/or may be configured to embed within the tissue wall 10 (e.g., between the distal surface 11 and the proximal surface 12). The side anchors 1105 may comprise free ends configured to extend away from the sensor body 1102 and/or configured to form an approximately 45-degree angle with the sensor body 1102. The side anchors 1105 may be configured to prevent the sensor body 1102 from becoming dislodged from the tissue wall 10.

The detachable tip 1106 is configured to become detached from the sensor body 1102, for example as shown in Figure 11. A tether 1109 interconnects the detachable tip 1106 and the sensor body 1102. In some examples, the tether 1109 may be at least partially composed of one or more shape memory alloys (e.g., Nitinol) and/or may be configured to naturally assume a bent and/or wavy form as shown in Figure 11. The tether 1109 is configured to direct the detachable tip 1106 away from the sensor body 1102 and/or towards the distal surface 11 of the tissue wall 10. The tether 1109 may be configured to apply a lateral force to the detachable tip 1106 such that the detachable tip 1106 and/or tether 1109 extend generally perpendicularly from the sensor body 1102 and/or such that a pointed end of the detachable tip 1106 is directed into contact with the distal surface 11 of the tissue wall 10. In some examples, the tether 1109 may be configured to drive the pointed end of the detachable tip 1106 into the distal surface 11 of the tissue wall 10 such that the pointed end punctures the distal surface 11.

The sensor device may be at least partially embedded in the tissue wall 10 and/or at least a portion of the sensor device may extend beyond the tissue wall 10. For example, at least the distal portion 1108 may extend beyond the distal surface 11 of the tissue wall 10. In some examples, a marker 1111 of the sensor device may be aligned with the distal surface 11 of the tissue wall 10, as shown in Figure 11.

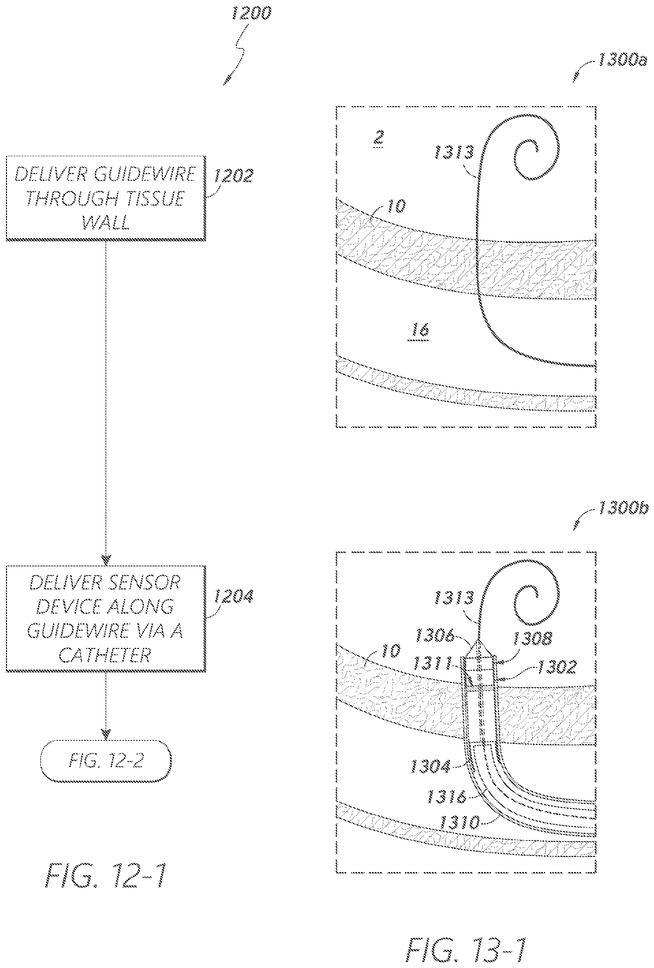

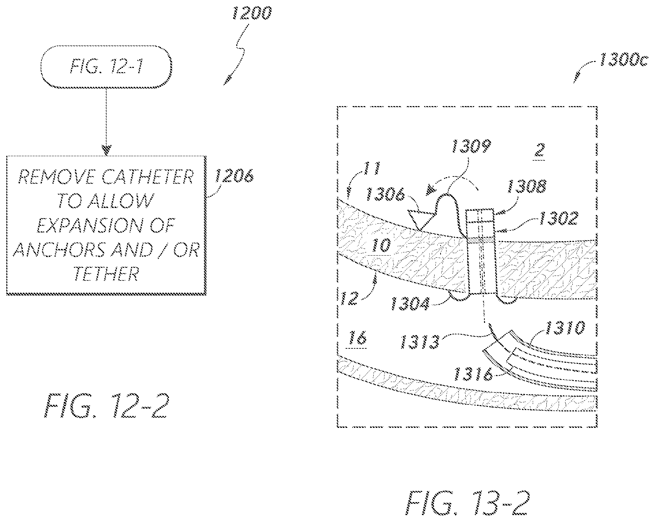

Figure 12 (Figures 12-1 and 12-2) provides a flowchart illustrating a process 1200 (not forming part of the presently claimed invention) including one or more steps for delivering one or more implants and/or sensors according to the presently claimed invention to target locations within a heart, in accordance with one or more examples. Figure 13 (Figures 13-1 and 13-2) provides images corresponding to steps of the process 1200 of Figure 12.

At step 1202, the process 1200 involves delivering a guidewire 1313 through a tissue wall 10 (e.g., a right atrium 2 wall), as illustrated in image 1300a of Figure 13-1. For example, the tissue wall 10 may separate the right atrium 2 from the coronary sinus 16. The guidewire 1313 may be delivered prior to delivery of one or more sensor devices and/or may be delivered in conjunction with the one or more sensor devices.

At step 1204, the process 1200 involves delivering a sensor device along the guidewire 1313 and/or at least partially within the tissue wall 10, as illustrated in image 1300b of Figure 13. In some examples, the sensor device may be delivered via a catheter 1310 and/or other shaft. A pusher 1316 and/or inner shaft may be used to press the sensor device towards a distal end of the catheter 1310.

The sensor device can comprise a sensor body 1302, a detachable tip 1306, one or more anchors 1304, and/or a marker 1311. The sensor device may further comprise at least one sensor component, which can be situated at a distal portion 1308 of the sensor body 1302 and/or elsewhere. The one or more anchors 1304 may be configured to assume a straightened and/or compressed form while within the catheter 1310. For example, the catheter 1310 may prevent the anchors 1304 from extending away from and/or towards the sensor body 1302. Similarly, the catheter 1310 may prevent the detachable tip 1306 from detaching from the sensor body 1302.

The sensor device may be extended through the tissue wall 10 until at least a portion of the sensor device extends beyond a distal side of the tissue wall 10 (e.g., into the right atrium 2). For example, at least the detachable tip 1306 and/or the distal portion 1308 of the sensor body 1302 may be situated at least partially within the right atrium 2 and/or beyond the tissue wall 10.

At step 1206, the process 1200 involves removing the catheter 1310 and/or guidewire 1313 to allow expansion and/or bending of the anchors 1304 and/or tether 1309 connecting the detachable tip 1306 to the sensor body 1302, as shown in image 1300c of Figure 13. In some examples, the tether 1309 may be shape set to direct the detachable tip 1306 away from the sensor body 1302 and/or to a side of the sensor body 1302. For example, the tether 1309 may be configured to naturally direct the detachable tip 1306 towards a distal side 11 of the tissue wall 10. The detachable tip 1306 may be configured to contact and/or puncture the distal side 11 of the tissue wall 10.

The one or more anchors 10 may similarly naturally bend in response to removal of the catheter 1310 and/or may be configured to contact and/or puncture a proximal side of the tissue wall 10. The sensor device may further comprise distal end anchors (not shown) configured to contact and/or puncture the distal side 11 of the tissue wall 10 and/or side anchors (not shown) configured to anchor against and/or embed within the tissue wall 10.

### Additional Examples

Depending on the example, certain acts, events, or functions of any of the processes or algorithms described herein can be performed in a different sequence, may be added, merged, or left out altogether. Thus, in certain examples, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain examples include, while other examples do not include, certain features, elements and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single example, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example. Thus, it is intended that the scope of the inventions herein disclosed and claimed below should not be limited by the particular examples described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A sensor implant device (700) comprising:
a sensor body (702) comprising at least a first sensor component;
one or more anchors (704, 705) coupled to the sensor body and configured to anchor the sensor body within a tissue wall;
a detachable tip (706); and
a tether (709) connecting the detachable tip to the sensor body;
**characterised in that**
the tether is shape set to direct the detachable tip away from the sensor body; and
**in that** the tether is configured to direct the detachable tip towards a surface of the tissue wall.

2. The sensor implant device of claim 1, wherein the tether is configured to direct the detachable tip
to a side of the sensor body.

3. The sensor implant device of claim 2, wherein the tether is composed a shape memory alloy.

4. The sensor implant device of any of claims 1-3, wherein the detachable tip has:
a) a conical shape with a pointed end, optionally wherein the pointed end is configured to pierce the tissue wall; or
b) a rounded end.

5. The sensor implant device of any of claims 1-4, wherein the detachable tip is configured to provide a leading end for the sensor body during delivery to the tissue wall,
optionally wherein the detachable tip comprises a flat base configured to lay flatly against a distal end of the sensor body.

6. The sensor implant device of any of claims 1-5, wherein the one or more anchors comprise one or more proximal end anchors (704, 804, 904) extending from a proximal end of the sensor body.

7. The sensor implant device of claim 6, wherein the one or more proximal end anchors (704) have semi-circular shapes that are concentric with the sensor body.

8. The sensor implant device of claim 6, wherein the one or more proximal end anchors (804, 904) have semi-circular shapes that extend along the sensor body towards a distal end of the sensor body.

9. The sensor implant device of any of claims 6 to 8, wherein the one or more anchors comprise three proximal end anchors.

10. The sensor implant device of any of claims 1-9, wherein the one or more anchors comprise one or more distal end anchors (905) extending from a distal end of the sensor body,
optionally wherein the one or more distal end anchors have semi-circular shapes that extend along the sensor body towards a proximal end of the sensor body.

11. The sensor implant device of claim 10, wherein the one or more anchors comprise three distal end anchors.

12. The sensor implant device of any of claims 1-11, wherein the one or more anchors comprise one or more side anchors (705) extending from a midsection of the sensor body,
optionally wherein:
a) each of the one or more side anchors comprises a free end configured to form an approximately 45-degree angle with the sensor body;
b) the one or more side anchors are configured to embed into and/or press against tissue within an opening through a tissue wall; and/or
c) the one or more side anchors are attached to the sensor body at a first side and are configured to swing freely away from the sensor body.

13. The sensor implant device of any of claims 1-12, wherein the one or more anchors comprise three side anchors.

14. The sensor implant device of any of claims 1-13, further comprising a marker (711) attached to the sensor body.

15. The sensor implant device of any of claims 1-14, wherein the sensor body comprises an inner lumen (713) configured to receive a guidewire (1013),
optionally wherein the detachable tip comprises a lumen (715) configured to be aligned with the inner lumen of the sensor body during delivery.

## Patentansprüche

1. Sensorimplantatvorrichtung (700) umfassend:
einen Sensorkörper (702) umfassend mindestens eine erste Sensorkomponente;
einen oder mehrere Anker (704, 705), die an den Sensorkörper gekoppelt sind und dazu ausgelegt sind, den Sensorkörper in einer Gewebewand zu verankern;
eine abnehmbare Spitze (706); und
ein Halteband (709), das die abnehmbare Spitze mit dem Sensorkörper verbindet;
**dadurch gekennzeichnet, dass**
das Halteband geformt ist, die abnehmbare Spitze von dem Sensorkörper weg zu lenken; und
dass das Halteband dazu ausgelegt ist, die abnehmbare Spitze in Richtung zu einer Oberfläche der Gewebewand zu lenken.

2. Sensorimplantatvorrichtung nach Anspruch 1, wobei das Halteband dazu ausgelegt ist, die abnehmbare Spitze zu einer Seite des Sensorkörpers zu lenken.

3. Sensorimplantatvorrichtung nach Anspruch 2, wobei das Halteband aus einer Formgedächtnislegierung besteht.

4. Sensorimplantatvorrichtung nach einem der Ansprüche 1-3, wobei die ablösbare Spitze aufweist:
a) eine konische Form mit einem spitzen Ende, gegebenenfalls wobei das spitze Ende dazu ausgelegt ist, die Gewebewand zu durchstechen; oder
b) ein abgerundetes Ende

5. Sensorimplantatvorrichtung nach einem der Ansprüche 1-4, wobei die abnehmbare Spitze dazu ausgelegt ist, ein vorderes Ende für den Sensorkörper während der Abgabe an die Gewebewand bereitzustellen,
gegebenenfalls wobei die abnehmbare Spitze eine flache Basis umfasst, die dazu ausgelegt ist, flach an einem distalen Ende des Sensorkörpers anzuliegen.

6. Sensorimplantatvorrichtung nach einem der Ansprüche 1-5, wobei der eine oder die mehreren Anker einen oder mehrere proximale Endanker (704, 804, 904) umfassen, die von einem proximalen Ende des Sensorkörpers ausgehen.

7. Sensorimplantatvorrichtung nach Anspruch 6, wobei der eine oder die mehreren proximalen Endanker (704) halbkreisförmige Formen aufweisen, die konzentrisch mit dem Sensorkörper sind.

8. Sensorimplantatvorrichtung nach Anspruch 6, wobei der eine oder die mehreren proximalen Endanker (804, 904) halbkreisförmige Formen aufweisen, die entlang des Sensorkörpers in Richtung zu einem distalen Ende des Sensorkörpers verlaufen.

9. Sensorimplantatvorrichtung nach einem der Ansprüche 6 bis 8, wobei der eine oder die mehreren Anker drei proximale Endanker umfassen.

10. Sensorimplantatvorrichtung nach einem der Ansprüche 1-9, wobei der eine oder die mehreren Anker einen oder mehrere distale Endanker (905) umfassen, die von einem distalen Ende des Sensorkörpers ausgehen, gegebenenfalls wobei der eine oder die mehreren distalen Endanker halbkreisförmige Formen aufweisen, die entlang des Sensorkörpers zu einem proximalen Ende des Sensorkörpers verlaufen.

11. Sensorimplantatvorrichtung nach Anspruch 10, wobei der eine oder die mehreren Anker drei distale Endanker umfassen.

12. Sensorimplantatvorrichtung nach einem der Ansprüche 1-11, wobei der eine oder die mehreren Anker einen oder mehrere Seitenanker (705) umfassen, die von einem Mittelabschnitt des Sensorkörpers ausgehen, gegebenenfalls wobei:
a) jeder des einen oder der mehreren Seitenanker ein freies Ende umfasst, das dazu ausgelegt ist, einen Winkel von etwa 45 Grad mit dem Sensorkörper zu bilden;
b) der eine oder die mehreren Seitenanker dazu ausgelegt sind, sich in Gewebe in einer Öffnung durch eine Gewebewand einzubetten und/oder dagegen zu drücken; und/oder
c) der eine oder die mehreren Seitenanker an einer ersten Seite an dem Sensorkörper angebracht sind und dazu ausgelegt sind, frei von dem Sensorkörper weg zu schwingen.

13. Sensorimplantatvorrichtung nach einem der Ansprüche 1-12, wobei der eine oder die mehreren Anker drei Seitenanker umfassen.

14. Sensorimplantatvorrichtung nach einem der Ansprüche 1-13, ferner umfassend einen Marker (711), der an dem Sensorkörper angebracht ist.

15. Sensorimplantatvorrichtung nach einem der Ansprüche 1-14, wobei der Sensorkörper ein Innenlumen (713) umfasst, dazu ausgelegt, einen Führungsdraht (1013) aufzunehmen,
gegebenenfalls wobei die abnehmbare Spitze ein Lumen (715) umfasst, dazu ausgelegt, während der Abgabe mit dem Innenlumen des Sensorkörpers ausgerichtet zu sein.

## Revendications

1. Dispositif implantable à capteur (700) comprenant :
un corps de capteur (702) comprenant au moins un premier composant capteur ;
un ou plusieurs ancrages (704, 705) couplés au corps de capteur et configurés pour ancrer le corps de capteur au sein d'une paroi tissulaire ;
un embout détachable (706) ; et
un lien (709) reliant l'embout détachable au corps de capteur ;
**caractérisé en ce que**
le lien est mis en forme pour diriger l'embout détachable à l'écart du corps de capteur ; et
**en ce que** le lien est configuré pour diriger l'embout détachable vers une surface de la paroi tissulaire.

2. Dispositif implantable à capteur selon la revendication 1, dans lequel le lien est configuré pour diriger l'embout détachable
vers un côté du corps de capteur.

3. Dispositif implantable à capteur selon la revendication 2, dans lequel le lien est composé d'un alliage à mémoire de forme.

4. Dispositif implantable à capteur selon l'une quelconque des revendications 1 à 3, dans lequel l'embout détachable présente :
a) une forme conique avec une extrémité pointue, facultativement l'extrémité pointue étant configurée pour percer la paroi tissulaire ; ou
b) une extrémité arrondie.

5. Dispositif implantable à capteur selon l'une quelconque des revendications 1 à 4, dans lequel l'embout détachable est configuré pour servir d'extrémité avant au corps de capteur pendant l'acheminement vers la paroi tissulaire,
facultativement dans lequel l'embout détachable comprend une base plate configurée pour reposer à plat contre une extrémité distale du corps de capteur.

6. Dispositif implantable à capteur selon l'une quelconque des revendications 1 à 5, dans lequel le ou les ancrages comprennent un ou plusieurs ancrages d'extrémité proximale (704, 804, 904) s'étendant depuis une extrémité proximale du corps de capteur.

7. Dispositif implantable à capteur selon la revendication 6, dans lequel le ou les ancrages d'extrémité proximale (704) présentent des formes semi-circulaires qui sont concentriques avec le corps de capteur.

8. Dispositif implantable à capteur selon la revendication 6, dans lequel le ou les ancrages d'extrémité proximale (804, 904) présentent des formes semi-circulaires qui s'étendent le long du corps de capteur vers une extrémité distale du corps de capteur.

9. Dispositif implantable à capteur selon l'une quelconque des revendications 6 à 8, dans lequel le ou les ancrages comprennent trois ancrages d'extrémité proximale.

10. Dispositif implantable à capteur selon l'une quelconque des revendications 1 à 9, dans lequel le ou les ancrages comprennent un ou plusieurs ancrages d'extrémité distale (905) s'étendant depuis une extrémité distale du corps de capteur,
facultativement dans lequel le ou les ancrages d'extrémité distale présentent des formes semi-circulaires qui s'étendent le long du corps de capteur vers une extrémité proximale du corps de capteur.

11. Dispositif implantable à capteur selon la revendication 10, dans lequel le ou les ancrages comprennent trois ancrages d'extrémité distale.

12. Dispositif implantable à capteur selon l'une quelconque des revendications 1 à 11, dans lequel le ou les ancrages comprennent un ou plusieurs ancrages latéraux (705) s'étendant depuis un tronçon médian du corps de capteur,
facultativement dans lequel :
a) le ou les ancrages latéraux comprennent chacun une extrémité libre configurée pour former un angle d'environ 45 degrés avec le corps de capteur ;
b) le ou les ancrages latéraux sont configurés pour s'enfoncer dans et/ou s'appuyer contre du tissu à l'intérieur d'une ouverture traversant une paroi tissulaire ; et/ou
c) le ou les ancrages latéraux sont fixés au corps de capteur au niveau d'un premier côté et sont configurés pour pivoter librement à l'écart du corps de capteur.

13. Dispositif implantable à capteur selon l'une quelconque des revendications 1 à 12, dans lequel le ou les ancrages comprennent trois ancrages latéraux.

14. Dispositif implantable à capteur selon l'une quelconque des revendications 1 à 13, comprenant en outre un marqueur (711) fixé au corps de capteur.

15. Dispositif implantable à capteur selon l'une quelconque des revendications 1 à 14, dans lequel le corps de capteur comprend une lumière interne (713) configurée pour recevoir un fil-guide (1013), facultativement dans lequel l'embout détachable comprend une lumière (715) configurée pour être alignée avec la lumière interne du corps de capteur pendant l'acheminement.
